# EUROPEAN PATENT APPLICATION

(11) **EP 4 483 818 A1**
(43) Date of publication of application: **01.01.2025**
(21) Application number: 24184922.3
(22) Date of filing: 27.06.2024
(51) Int. Cl.: A61B 17/15, A61B 17/17, A61B 34/20

(54) **DEVICES AND SYSTEMS FOR DYNAMIC SURGICAL GUIDES**

(30) Priority: 29.06.2023 US 202363510936 P; 21.06.2024 US 202418749820
(71) Applicant: Wright Medical Technology, Inc., Memphis, TN 38117 (US)
(72) Inventor: KORMAN, Zachary, St. Louis, 63109 (US); BAILEY, Erroll, Atlanta, 30328 (US); STEINLAUF, Steven, Weston, 33327 (US); BEHRENS, Andrew, Polk City, 50226 (US); MASSARO, Joey, Ridgefield, 07657 (US); SEIDL, Amanda, Memphis, 38103 (US); STEMNISKI, Paul, Memphis, 38122 (US); PATERSON, Hannah, Memphis, 38104 (US)
(74) Representative: Zimmermann & Partner Patentanwälte mbB

(57) **Abstract**

In some embodiments, a surgical guide may include a body having a first portion with a first ridge and a second ridge, and a second portion with a first ridge and a second ridge.
The surgical guide may also include an insert disposed between the first ridge and the second ridge of the first portion and the first ridge and second ridge of the second portion. The insert may be configured to rotate relative to the body. The surgical guide may also include a guide aperture disposed within the insert that is sized to receive a surgical instrument. Other surgical guides and methods of using surgical guides are also disclosed.

## Description

### CROSS-REFERENCE TO RELATED APPLICATION

This application claims priority under 35 U.S.C. § 119(e) to U.S. Provisional Application No. 63/510,936, filed June 29, 2023, the entirety of which is incorporated by reference herein.

### FIELD OF THE INVENTION

Surgical guides, systems and methods are provided that are suitable for dynamic intraoperative adjustment by a surgeon.

### BACKGROUND OF THE INVENTION

Surgical procedures, such as osteotomies, typically include a guide designed to enable a surgeon to make accurate and precise bone resections to correct a deformity. Often, a surgical guide will have a fixed shape and will be removable so as to change its location or to employ different cut shapes. This aspect requires additional time during surgery and may interfere with the surgical techniques being employed by the surgical team.

Pre-sized and patient-specific cutting guides are designed based upon average patient size or pre-operative imaging of the surgical site from which the surgeon prepares a pre-operative plan. Conventional cutting guides are designed to have pre-defined shapes such as a transverse, Wedge, or Chevron depending upon the surgical requirements for the particular osteotomy. The development of a pre-operative plan including the design of an appropriate cutting guide can be time consuming and expensive. When the final cutting guide is manufactured and a part of the surgical kit, there is little possibility for design deviations if the pre-operative plan evolves. During surgery, if a surgeon determines that the pre-operative plan may need to change as a result of unforeseen circumstances, the only manner in which the surgeon may deviate the pre-operative plan is by hand, i.e., without guidance from the pre-sized or patient-specific cutting guide.

For minimally-invasive procedures related to the foot, creating accurate osteotomies in the calcaneus is difficult due to the size of the bone and the need to make multiple cuts to complete a single portion of the osteotomy. Furthermore, duration of cutting and depth of the cut may lead to higher thermal energy being generated at the surgical site which requires dissipation so as to prevent necrosis. Conventional solutions to this problem include altering the size of the cutting instrument and using "water pick" irrigation. However, these techniques often lead to inaccurate and jagged cuts in the bone and often risk insufficient cooling. Accordingly, there has been a long felt need in the field for improved surgical guides, systems, and methods that provide for dynamic variation and precise bone resections.

### SUMMARY

In some embodiments, a surgical guide may include a body having a first portion with a first ridge and a second ridge, and a second portion with a first ridge and a second ridge. The surgical guide may also include an insert disposed between the first ridge and the second ridge of the first portion and the first ridge and second ridge of the second portion. The insert may be configured to rotate relative to the body. The surgical guide may also include a guide aperture disposed within the insert that is sized to receive a surgical instrument.

In some embodiments, a surgical system may include a surgical guide. The surgical guide may include a body having a first portion with a first ridge and a second ridge, and a second portion with a first ridge and a second ridge. The surgical guide may also include an insert disposed between the first ridge and the second ridge of the first portion and the first ridge and second ridge of the second portion. The insert may be configured to rotate relative to the body. The surgical guide may also include a guide aperture disposed within the insert that is sized to receive a surgical instrument. The surgical system may also include a surgical navigation system having a computing device with a processor and a display configured to display intraoperative adjustments of the guide aperture.

In some embodiments, a surgical guide may include a body having a first portion with a first ridge and a second ridge, and a second portion with a first ridge and a second ridge. The surgical guide may also include an insert having a raised portion. The insert may be disposed between the first ridge and the second ridge of the first portion and the first ridge and second ridge of the second portion. The insert may be configured to rotate relative to the body. The surgical guide may also include a guide aperture disposed within the insert that is sized to receive a surgical instrument.

In some embodiments, a surgical guide may include a body having a raised portion. The body may define one or more holding features configured to receive a fixation element. The body may also include an aperture that is sized and configured to receive a sight and an alignment ring. The surgical guide may also include a guide aperture disposed within the body that is sized to receive a surgical instrument. The surgical guide may also include an irrigation port configured to provide irrigation to a surgical site.

In some embodiments, a method of using a surgical guide may include coupling the surgical guide to a bone with one or more first fixation elements. The method may also include adjusting, to a first angle, an insert disposed within a body of the surgical guide to align a guide aperture with a desired surgical site on the bone. The method may also include locking the insert. The method may also include inserting a surgical instrument into the guide aperture. The method may also include making a first cut of the bone with the surgical instrument.

In some embodiments, a method of using a surgical system includes determining a reference point for a surgical procedure based at least in part on a surgical navigation system. The method may also include coupling a surgical guide to a bone with one or more first fixation elements. The method may also include adjusting, to a first angle, an insert disposed within the surgical guide to align a guide aperture with a desired surgical site on the bone based at least in part on the surgical navigation system. The method may also include locking the insert. The method may also include inserting a surgical instrument into the guide aperture. The method may also include making a first cut of the bone with the surgical instrument.

Further disclosed herein is aa surgical guide that may include a body having a first portion with a first ridge and a second ridge, and a second portion with a first ridge and a second ridge, wherein the surgical guide may also include an insert disposed between the first ridge and the second ridge of the first portion and the first ridge and second ridge of the second portion, wherein the insert may be configured to rotate relative to the body, and wherein the surgical guide may also include a guide aperture disposed within the insert that is sized to receive a surgical instrument.

### BRIEF DESCRIPTION OF THE DRAWINGS

The features and advantages of the invention will be more fully disclosed in, or rendered obvious by the following detailed exemplary descriptions of embodiments. The detailed descriptions of these exemplary embodiments are to be considered together with the accompanying drawings wherein like numbers refer to like parts and further wherein:
FIG. 1 illustrates an isometric view of a first surgical guide in accordance with some embodiments;
FIG. 2 illustrates an exploded view of a first surgical guide in accordance with some embodiments;
FIG. 3 illustrates a top down view of a first surgical guide in accordance with some embodiments;
FIG. 4 illustrates a cross-sectional view of a first surgical guide along axis 4-4 shown in FIG. 3 in accordance with some embodiments;
FIG. 5 illustrates an isometric view of a second surgical guide in accordance with some embodiments;
FIG. 6 illustrates an exploded view of a second surgical guide in accordance with some embodiments;
FIG. 7 illustrates a top down view of a second surgical guide in accordance with some embodiments;
FIG. 8 illustrates a cross-sectional view of a second surgical guide along axis 8-8 shown in FIG. 7 in accordance with some embodiments;
FIG. 9 illustrates a side view of a second surgical guide in accordance with some embodiments;
FIG. 10 illustrates an isometric view of a third surgical guide in accordance with some embodiments;
FIG. 11 illustrates an exploded view of a third surgical guide in accordance with some embodiments;
FIG. 12 illustrates an isometric view of a top portion of a third surgical guide in accordance with some embodiments;
FIG. 13 illustrates a top down view of a third surgical guide in accordance with some embodiments;
FIG. 14 illustrates a side view of a third surgical guide in accordance with some embodiments;
FIG. 15 illustrates a cross-sectional view of a third surgical guide along axis 15-15 shown in FIG. 13 in accordance with some embodiments;
FIG. 16 illustrates an isometric view of a fourth surgical guide in accordance with some embodiments;
FIG. 17 illustrates an exploded view of a fourth surgical guide in accordance with some embodiments;
FIG. 18 illustrates a top down view of a fourth surgical guide in accordance with some embodiments;
FIG. 19 illustrates a cross-sectional view of a fourth surgical guide along axis 19-19 shown in FIG. 18 in accordance with some embodiments;
FIG. 20 illustrates an isometric view of a fifth surgical guide in accordance with some embodiments;
FIG. 21 illustrates an exploded view of a fifth surgical guide in accordance with some embodiments;
FIG. 22 illustrates a top down view of a fifth surgical guide in accordance with some embodiments;
FIG. 23 illustrates a side view of a fifth surgical guide in accordance with some embodiments;
FIG. 24 illustrates an isometric view of a sixth surgical guide in accordance with some embodiments;
FIG. 25 illustrates an exploded view of a sixth surgical guide in accordance with some embodiments;
FIG. 26 illustrates a top down view of a sixth surgical guide in accordance with some embodiments;
FIG. 27A illustrates a first side view of a sixth surgical guide in accordance with some embodiments;
FIG. 27B illustrates a second side view of a sixth surgical guide in accordance with some embodiments;
FIG. 28 illustrates an isometric view of a seventh surgical guide in accordance with some embodiments;
FIG. 29 illustrates an exploded view of a seventh surgical guide in accordance with some embodiments;
FIG. 30 illustrates a top down view of a seventh surgical guide in accordance with some embodiments;
FIG. 31 illustrates a side view of a seventh surgical guide in accordance with some embodiments;
FIG. 32 illustrates an example of a surgical navigation system in accordance with some embodiments;
FIG. 33 illustrates an example of a display in accordance with some embodiments;
FIG. 34 illustrates a first method of using a surgical guide during a procedure in accordance with some embodiments; and
FIG. 35 illustrates a second method of using a surgical guide during a procedure in accordance with some embodiments.

### DETAILED DESCRIPTION

This description of the exemplary embodiments is intended to be read in connection with the accompanying drawings, which are to be considered part of the entire written description. It should be understood, however, that the present disclosure is not intended to be limited to the particular forms disclosed and that the drawings are not necessarily shown to scale. Rather, the present disclosure covers all modifications, equivalents, and alternatives that fall within the spirit and scope of these exemplary embodiments. In the description, relative terms such as "lower," "upper," "horizontal," "vertical," "above," "below," "up," "down," "top," and "bottom" as well as derivatives thereof (e.g., "horizontally," "downwardly," "upwardly," etc.) should be construed to refer to the orientation as then described or as shown in the drawing under discussion. These relative terms are for convenience of description and do not require that the apparatus be constructed or operated in a particular orientation. Terms concerning attachments, coupling and the like, such as "connected" and "interconnected," refer to a relationship wherein structures are secured or attached to one another either directly or indirectly through intervening structures, as well as both movable or rigid attachments or relationships, unless expressly described otherwise. The terms "couple," "coupled," "operatively coupled," "operatively connected," and the like should be broadly understood to refer to connecting devices or components together either mechanically, or otherwise, such that the connection allows the pertinent devices or components to operate with each other as intended by virtue of that relationship.

The guides, systems and methods being disclosed allow a surgeon to intraoperatively adjust a guide during a procedure so as to provide the surgeon with the ability to adjust the procedure without resorting to "free-hand" resection. The guides, systems, and methods may also use a surgical navigation system to aid the surgeon in dynamically aligning a guide for precise cuts in accordance with a pre-surgical plan. One example of such surgical navigation system is described in U.S. Provisional Patent Application No. 63/371,095 filed on August 11, 2022, entitled "Devices, Systems and Methods for User Guidance in Surgical Navigation", the entirety of which is incorporated herein by reference.

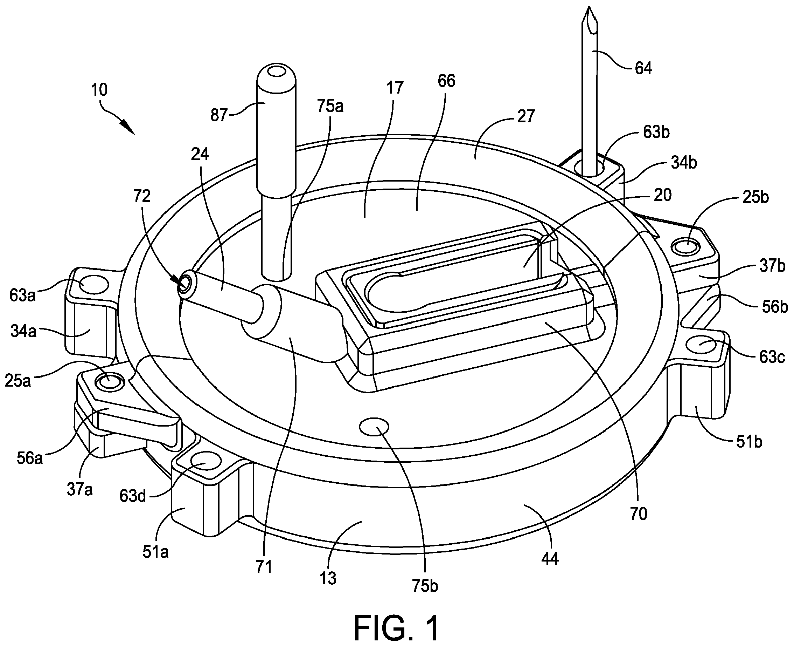

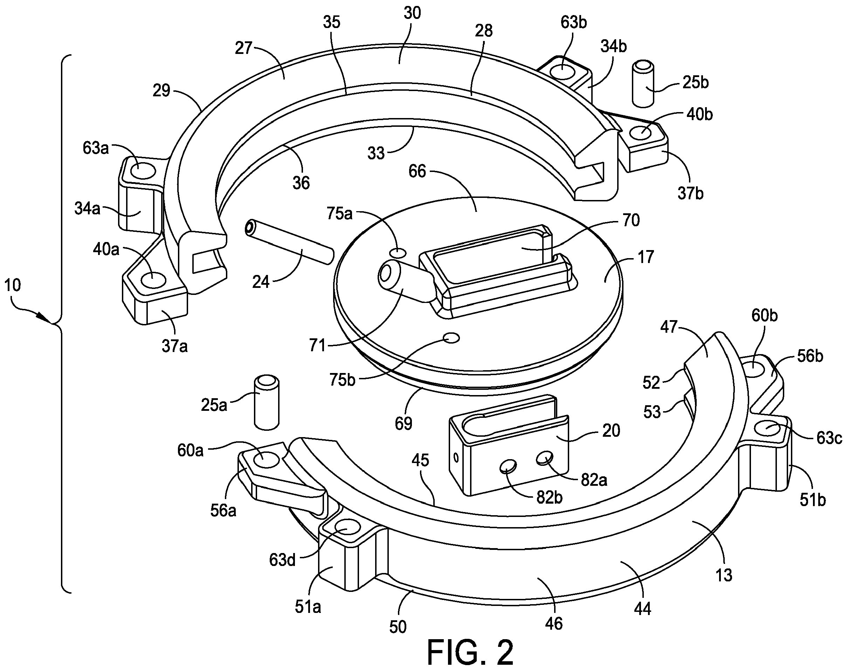

Turning now to FIGS. 1-4, a first surgical guide 10 has a body 13, an insert 17, a guide aperture 20, an irrigation port 24, and two pins 25a-b. Body 13 has a left portion 27 with an inner edge 28, an outer edge 29, a top surface 30, and a bottom surface 33. Left portion 27 defines at least one first protrusion 34a-b on the outer edge 29 of the left portion 27. Inner edge 28 defines a first ridge 35 and a second ridge 36. Left portion 27 of body 13 also has at least one securing member 37a-b disposed on the outer edge 29 of left portion 27 that defines a first passage 40a-b through left portion 27. Body 13 also has a right portion 44 with an inner edge 45, an outer edge 46, a top surface 47, and a bottom surface 50. Right portion 44 defines at least one second protrusion 51a-b on the outer edge 46 of the right portion 44. Inner edge 45 of right portion 44 defines a first ridge 52 and a second ridge 53. Right portion 44 of body 13 has at least one securing member 56a-b disposed on the outer edge 46 of right portion 44 that defines a second passage 60a-b through right portion 44.

Passage 40a of securing member 37a and passage 60a of securing member 56a are configured to align with one another thus defining a first void 61a in body 13. Pin 25a is sized to be received within first void 61a. Additionally, passage 40b of securing member 37b and passage 60b of securing member 56b are configured to align with one another defining a second void 61b through body 13. Pin 25b is sized to be received within second void 61b. Pin 25a located within first void 61a and pin 25b located within second void 61b couple left portion 27 to right portion 44.

Additionally, first protrusions 34a-b disposed on the left portion 27 and second protrusions 51a-b disposed on right portion 44 define channels 63a-d. It should be understood that body 13 may define one or more channels 63a-d depending on the number of first protrusions 34a-b and second protrusions 51a-b. Channels 63a-d are configured to receive one or more fixation elements 64 to couple the body 13 to one or more bones of a patient, e.g., k-wires, pins, screws or other similar structures. For example, one or more of the fixation elements 64 can be inserted through the one or more channels 63a-d and inserted into the bone, fixing the surgical guide 10 to the patient.

In some embodiments, the shape of body 13 may be circular, rectangular, square, or oblong. Additionally, the size and shape of body 13, and particularly bottom surface 33 of left portion 27 and bottom surface 50 of right portion 44, may be formed so as to have a patient-specific surface based on pre-operative fitting. The size and shape of body 13, and particularly bottom surface 33 of left portion 27 and bottom surface 50 of right portion 44, may also be formed so as to be patient-specific based on imaging of the surgical site. While in some embodiments body 13 may be one piece, in other embodiments, body 13 may be formed of two pieces (i.e., a separate left portion 27 and a separate right portion 44). In some embodiments, surgical guide 10 may be formed from a medical-grade material that is capable of being 3D printed (e.g., additively manufactured), such as ABS (acrylonitrile butadiene styrene), PLA (polylactic acid), PETG (polyethylene terephthalate glycol), nylon, TPU (thermoplastic polyurethane), resin, and other suitable thermoplastics and thermosetting plastics. However, surgical guide 10 may be formed from other materials, including metals, ceramics, and other materials that are suitable for use in surgery as will be understood by one of ordinary skill in the art. In some embodiments, surgical guide 10 may be machined and/or formed using an additive manufacturing process, such as electron beam melting (EBM) or direct metal laser sintering (DMLS), to list only a few possibilities.

Still referring to FIGS. 1-4, first surgical guide 10 also includes insert 17 having a top surface 66, a bottom surface 69, a receiving surface 70, and a port 71. Insert 17 is disposed within body 13 between first ridge 35 and second ridge 36 of left portion 27 and between first ridge 52 and second ridge 53 of right portion 44. Insert 17 defines a receiving surface 70 disposed through insert 17 between top surface 66 and bottom surface 69. Insert 17 also defines port 71 disposed on top surface 66. Receiving surface 70 is configured to receive guide aperture 20. In some embodiments, insert 17 includes guide aperture 20, removing the need for receiving surface 70. Port 71 is configured to receive irrigation port 24. Guide aperture 20 and irrigation port 24 define a canal 72 that extends from irrigation port 24 into guide aperture 20. Insert 17 also defines one or more holes 75a-b that communicate between top surface 66 and bottom surface 69 via insert 17. In some embodiments, the shape of insert 17 may be circular, rectangular, square, etc. in order to match the shape of body 13. Insert 17 is often configured to move (i.e., rotate or translate) with respect to body 13. In some embodiments, insert 17 may be configured to rotate 360 degrees. In some embodiments, body 13 and insert 17 are formed from different materials.

Guide aperture 20 is sized and configured to receive a surgical instrument 81, such as a drill, burr, etc. For example, guide aperture 20 may be a slot, such as a substantially rectangular slot, sized and configured to receive surgical instrument 81, such as a burr, to cut bone during a surgical procedure. In some embodiments, guide aperture 20 may be a circular or oval opening, sized and configured to receive surgical instrument 81, such as a drill, to drill into the bone to a pre-determined depth. In some embodiments, guide aperture 20 includes one or more manufacturing features 82a-b. Guide aperture 20 may be made of metal to protect insert 17 from abrasion due to its interaction with surgical instrument 81. In other embodiments, guide aperture 20 may be formed of a polymer or other radiolucent material to be able to precisely see the location of surgical instrument 81 through intraoperative imaging.

Holes 75a-b defined by insert 17 are sized to receive fixation elements 64 that lock insert 17 so as to place guide aperture 20 at a desired angle. For example, fixation elements 64 can include k-wire, pins, screws or other similar structures. In other embodiments, a locking device 84 may be used to fixate guide aperture 20 of insert 17 at a fixed angle rather than using one or more fixation elements 64 through holes 75a-b.

Irrigation port 24 allows liquid (i.e., water, saline, etc.) to be introduced to the surgical site for cooling during a procedure. The liquid passing through irrigation port 24 may also be used to remove bone or tissue debris caused by the cutting or drilling. For example, liquid through canal 72 and manufacturing features 82a-b may be used to remove bone or tissue debris from guide aperture 20. In some embodiments, irrigation port 24 may also be configured to receive a suction hose to provide suction to the surgical site in order to remove bone or tissue debris. Irrigation port 24 may also be configured to act as a handle that can be gripped or engaged with a tool, such as pliers, in order to facilitate removal of surgical guide 10 from bone. In other embodiments, surgical guide 10 may include a handle 87 disposed within one of the holes 75a-b or from an alternate additional hole.

Surgical guide 10 may also include one or more radiopaque markers 90 disposed within insert 17. Radiopaque markers 90 may be used as reference markers to indicate a change in orientation of guide aperture 20 using a camera, fluoroscopy, x-ray, CT image. This allows a surgeon to intraoperatively move insert 17 to align guide aperture 20 at a desired angle. In some embodiments, radiopaque markers 90 may also be used in conjunction with a surgical navigation system discussed below, which may be configured to display the orientation of guide aperture 20 on a display. For example, radiopaque markers 90 may provide the surgical navigation system with markers that can be tracked as insert 17 is moved (i.e., rotated or translated). In some embodiments, pins 25a-b may also be used as markers, similar to radiopaque markers 90, that communicate the position of body 13 to the surgical navigation system.

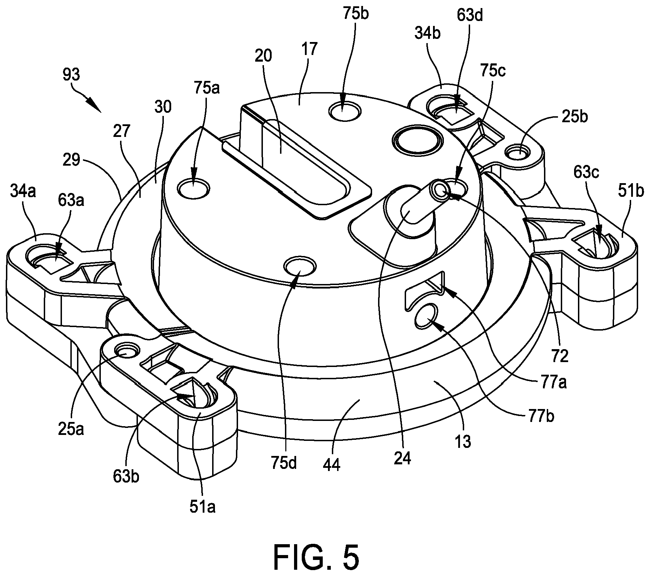

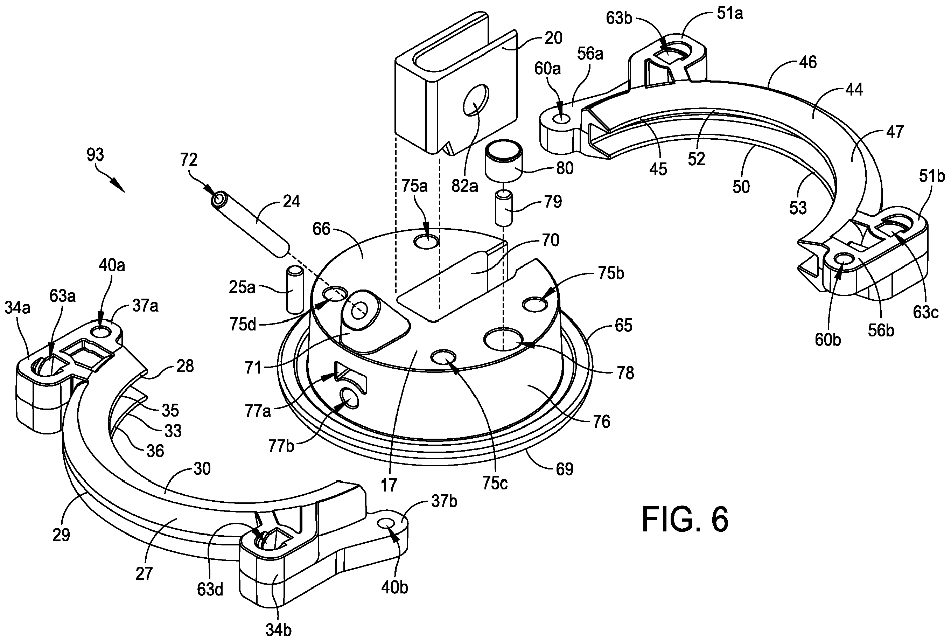

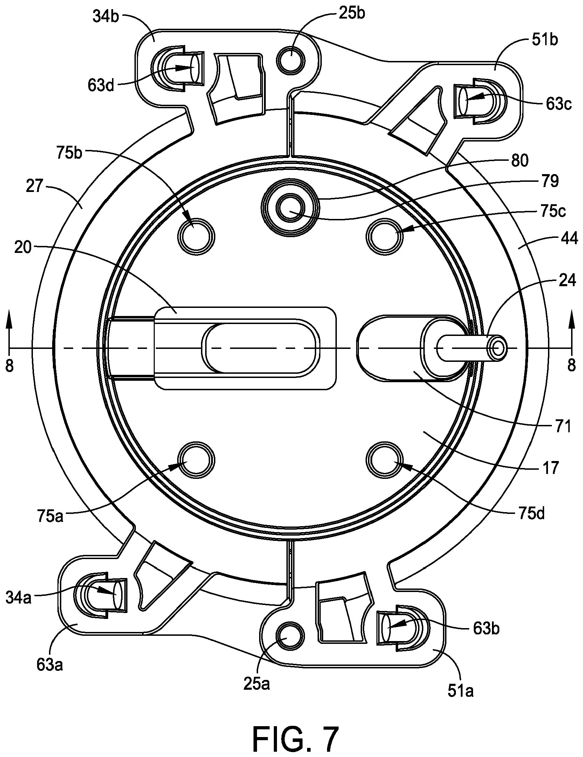

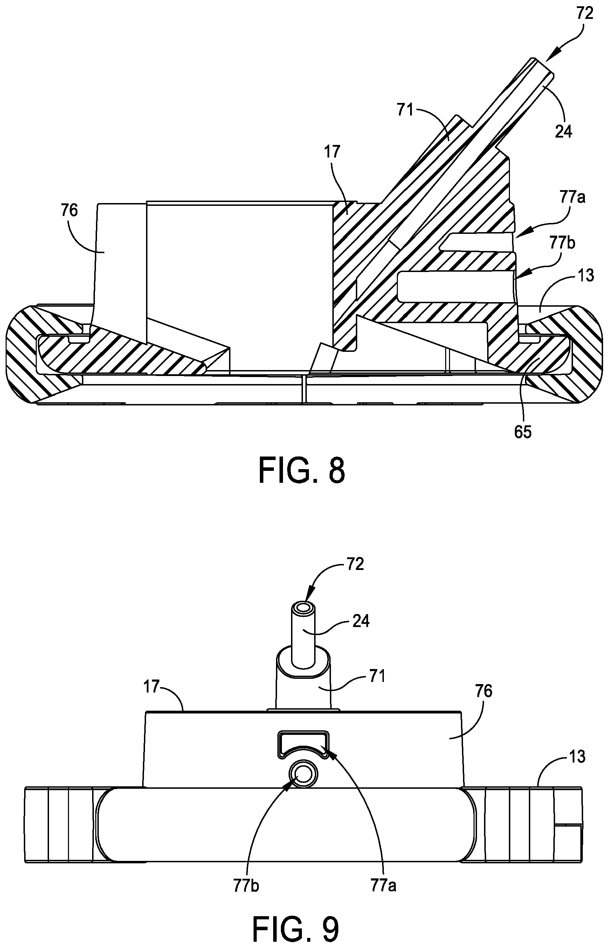

Referring now to FIGS. 5-9, a second surgical guide 93 has a body 13, an insert 17, a guide aperture 20, an irrigation port 24, and two pins 25a-b. Body 13 has a left portion 27 with an inner edge 28, an outer edge 29, a top surface 30, and a bottom surface 33. Left portion 27 defines at least one first protrusion 34a-b on the outer edge 29 of the left portion 27. Inner edge 28 defines a first ridge 35 and a second ridge 36. Left portion 27 of body 13 also has at least one securing member 37a-b disposed on the outer edge 29 of left portion 27 that defines a first passage 40a-b through left portion 27. Body 13 also has a right portion 44 with an inner edge 45, an outer edge 46, a top surface 47, and a bottom surface 50. Right portion 44 defines at least one second protrusion 5 1a-b on the outer edge 46 of the right portion 44. Inner edge 45 of right portion 44 defines a first ridge 52 and a second ridge 53. Right portion 44 of body 13 has at least one securing member 56a-b disposed on the outer edge 46 of right portion 44 that defines a second passage 60a-b through right portion 44. Passage 40a of securing member 37a and passage 60a of securing member 56a are configured to align and receive pin 25a. Additionally, passage 40b of securing member 37b and passage 60b of securing member 56b are configured to align and receive pin 25b. Pins 25a-b couple left portion 27 to right portion 44.

In some embodiments, first protrusions 34a-b disposed on the left portion 27 and second protrusions 51a-b disposed on right portion 44 define channels 63a-d. It should be understood that body 13 may define one or more channels 63a-d depending on the number of first protrusions 34a-b and second protrusions 51a-b. Channels 63a-d are configured to receive one or more fixation elements, such as fixation element 64 illustrated in FIG. 1, to couple the body 13 to one or more bones of a patient, e.g., k-wires, pins, screws or other similar structures. For example, one or more of the fixation elements can be inserted through the one or more channels 63a-d and inserted into the bone, fixing the surgical guide 93 to the patient. In other embodiments, the first protrusions 34a-b and the second protrusions 51a-b may define channels 63a-d at an angle such that fixation elements are inserted and disposed within channels 63a-d at some angle with reference to body 13.

In many embodiments, the shape of body 13 may be circular, rectangular, square, or oblong. Additionally, the size and shape of body 13, and particularly bottom surface 33 of left portion 27 and bottom surface 50 of right portion 44, may be formed so as to have a patient-specific surface based on pre-operative fitting. The size and shape of body 13, and particularly bottom surface 33 of left portion 27 and bottom surface 50 of right portion 44, may also be formed so as to be patient-specific based on imaging of the surgical site. While in some embodiments body 13 may be one piece, in other embodiments, body 13 may be formed of two pieces (i.e., a separate left portion 27 and a separate right portion 44). In some embodiments, surgical guide 93 may be formed from a medical-grade material that is capable of being 3D printed (e.g., additively manufactured), such as ABS (acrylonitrile butadiene styrene), PLA (polylactic acid), PETG (polyethylene terephthalate glycol), nylon, TPU (thermoplastic polyurethane), resin, and other suitable thermoplastics and thermosetting plastics. However, surgical guide 93 may be formed from other materials, including metals, ceramics, and other materials that are suitable for use in surgery as will be understood by one of ordinary skill in the art. In some embodiments, surgical guide 93 may be machined and/or formed using an additive manufacturing process, such as electron beam melting (EBM) or direct metal laser sintering (DMLS), to list only a few possibilities.

Still referring to FIGS. 5-9, surgical guide 93 also includes insert 17 having a ring 65, a top surface 66, a bottom surface 69, a receiving surface 70, and a port 71. Ring 65 of insert 17 is disposed within body 13 between first ridge 35 and second ridge 36 of left portion 27 and between first ridge 52 and second ridge 53 of right portion 44. Insert 17 defines a receiving surface 70 disposed through insert 17 between top surface 66 and bottom surface 69. Insert 17 also defines port 71 disposed on top surface 66. Receiving surface 70 is configured to receive guide aperture 20. In some embodiments, insert 17 includes guide aperture 20, removing the need for receiving surface 70. Port 71 is configured to receive irrigation port 24. Guide aperture 20 and irrigation port 24 define a canal 72 that extends from irrigation port 24 into guide aperture 20. Insert 17 also defines one or more holes 75a-d that communicate between top surface 66 and bottom surface 69 via insert 17. In some embodiments, the shape of insert 17 may be circular, rectangular, square, etc. in order to match the shape of body 13. Insert 17 is often configured to move (i.e., rotate or translate) with respect to body 13. In some embodiments, insert 17 may be configured to rotate 360 degrees. In some embodiments, body 13 and insert 17 are formed from different materials. In some embodiments, insert 17 includes a raised portion 76 above body 13 making it easier to work with insert 17 (i.e., rotate, translate, etc). Raised portion 76 defines one or more holding features 77a-b. Holding features 77a-b are sized and configured to receive one or more fixation elements, such as fixation element 64 illustrated in FIG. 1, to lock insert 17 and prevent insert 17 from rotating. In some embodiments, insert 17 defines an aperture 78 that is sized and configured to receive a sight 79 and an alignment ring 80. Sight 79 and alignment ring 80 are used to reduce parallax error and verify that surgical guide 93 and a surgical instrument are aligned. For example, when surgical guide 93 and a surgical instrument are correctly aligned, a surgeon will see sight 79 in the middle of alignment ring 80. When surgical guide 93 and a surgical instrument are misaligned, sight 79 will no longer be centered in alignment ring 80 and may appear to fully or partially disappear from view.

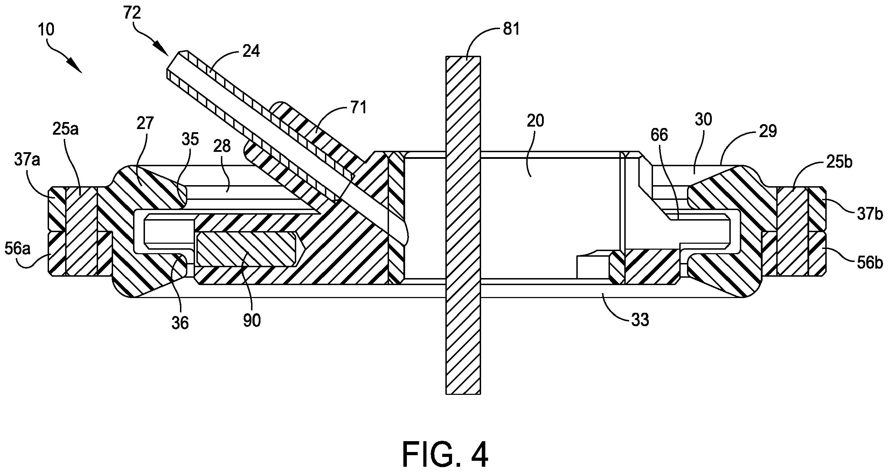

Guide aperture 20 is sized and configured to receive a surgical instrument, such as surgical instrument 81 illustrated in FIG. 4, which may be a drill, burr, etc. For example, guide aperture 20 may be a slot, such as a substantially rectangular slot, sized and configured to receive the surgical instrument, such as a burr, to cut bone during a surgical procedure. In some embodiments, guide aperture 20 may be a circular or oval opening, sized and configured to receive a surgical instrument, such as a drill, to drill into the bone to a pre-determined depth. In some embodiments, guide aperture 20 includes one or more manufacturing features 82a. Guide aperture 20 may be made of metal to protect insert 17 from abrasion due to its interaction with a surgical instrument. In other embodiments, guide aperture 20 may be formed of a polymer or other radiolucent material to be able to precisely see the location of a surgical instrument through intraoperative imaging.

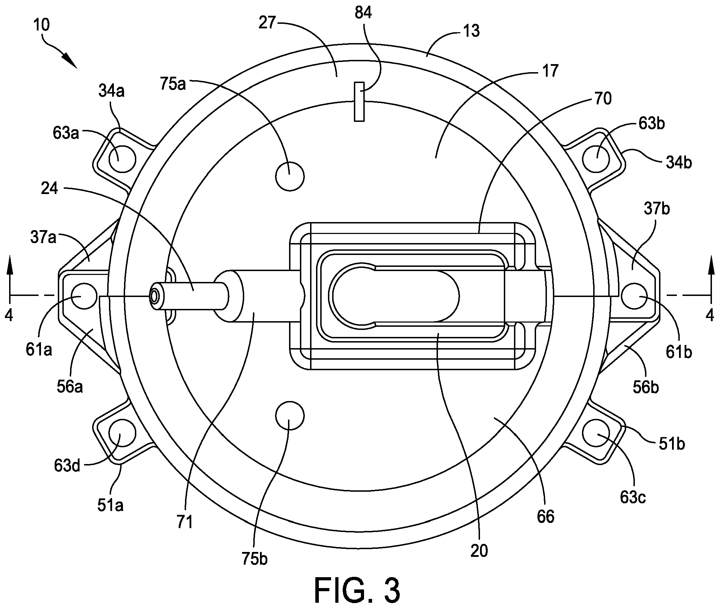

Holes 75a-b defined by insert 17 are sized to receive fixation elements that lock insert 17 so as to place guide aperture 20 at a desired angle. For example, fixation elements can include k-wire, pins, screws or other similar structures. In other embodiments, a locking device, such as locking device 84 illustrated in FIG. 3, may be used to fixate guide aperture 20 of insert 17 at a fixed angle rather than using one or more fixation elements through holes 75a-d or holding features 77a-b.

Irrigation port 24 allows liquid (i.e., water, saline, etc.) to be introduced to the surgical site for cooling during a procedure. The liquid passing through irrigation port 24 may also be used to remove bone or tissue debris caused by the cutting or drilling. For example, liquid through canal 72 and manufacturing feature 82a may be used to remove bone or tissue debris from guide aperture 20. In some embodiments, irrigation port 24 may also be configured to receive a suction hose to provide suction to the surgical site in order to remove bone or tissue debris. Irrigation port 24 may also be configured to act as a handle that can be gripped or engaged with a tool, such as pliers, in order to facilitate removal of surgical guide 93 from bone. In other embodiments, surgical guide 93 may include a handle, such as handle 87 illustrated in FIG. 1, disposed within one of the holes 75a-b or from an alternate additional hole.

Surgical guide 93 may also include one or more radiopaque markers disposed within insert 17, such as radiopaque marker 90 illustrated in FIG. 4. Radiopaque markers may be used as reference markers to indicate a change in orientation of guide aperture 20 using a camera, fluoroscopy, x-ray, CT image. This allows a surgeon to intraoperatively move insert 17 to align guide aperture 20 at a desired angle. In some embodiments, radiopaque markers may also be used in conjunction with a surgical navigation system discussed below, which may be configured to display the orientation of guide aperture 20 on a display. For example, radiopaque markers may provide the surgical navigation system with markers that can be tracked as insert 17 is moved (i.e., rotated or translated). In some embodiments, pins 25a-b may also be used as markers, similar to radiopaque markers, that communicate the position of body 13 to the surgical navigation system. In some embodiments, sight 79 and alignment ring 80 may also be used as markers, similar to radiopaque markers, that communicate the orientation of body 13 to the surgical navigation system.

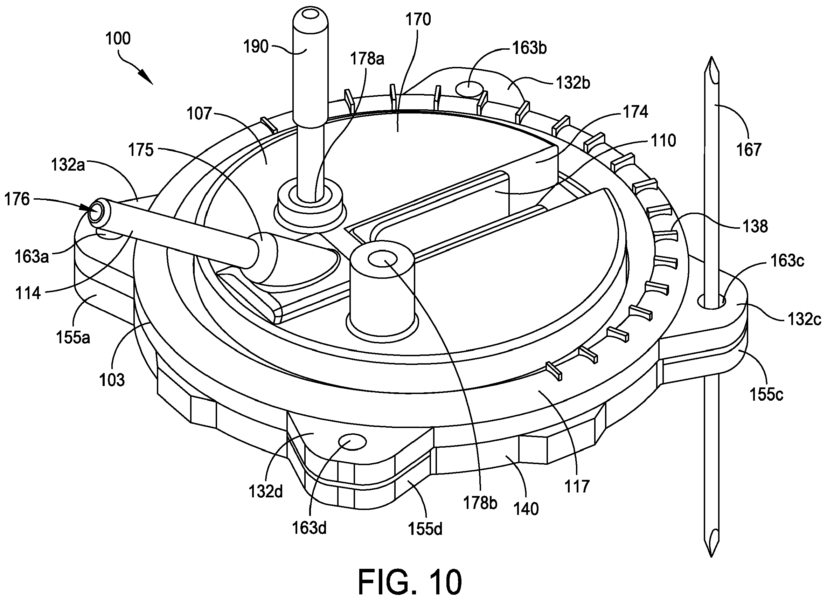

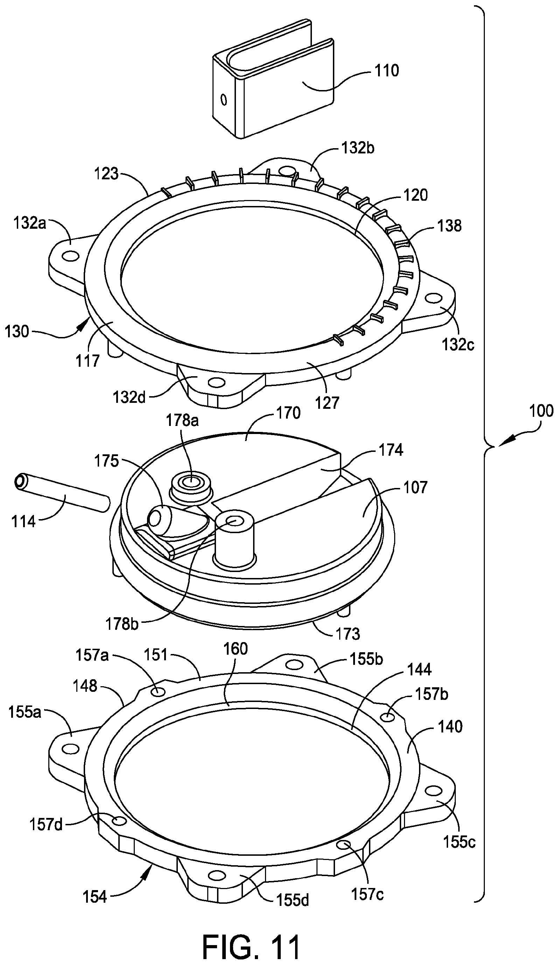

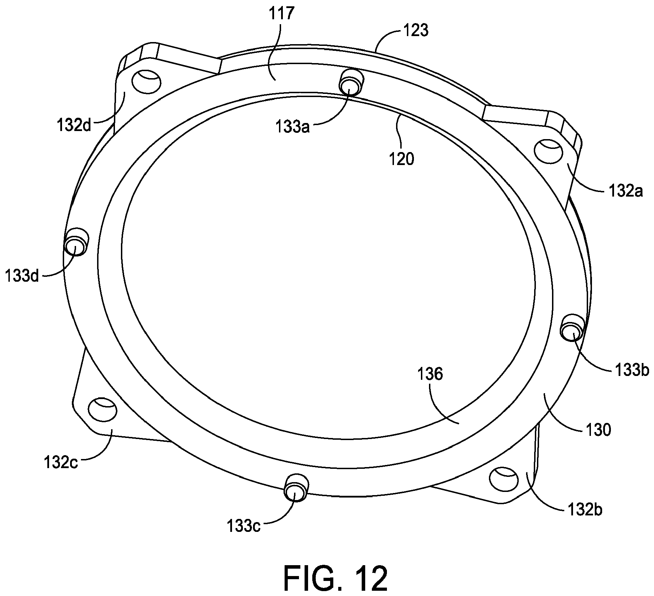

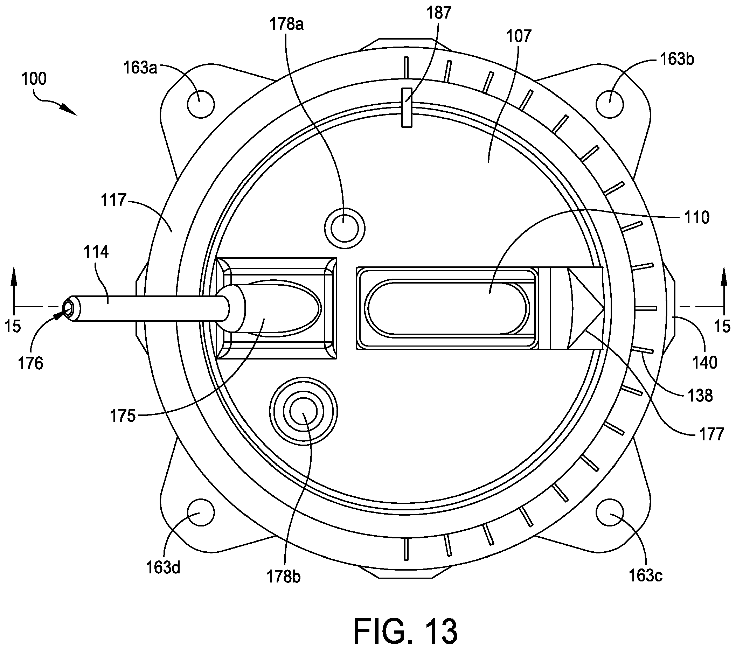

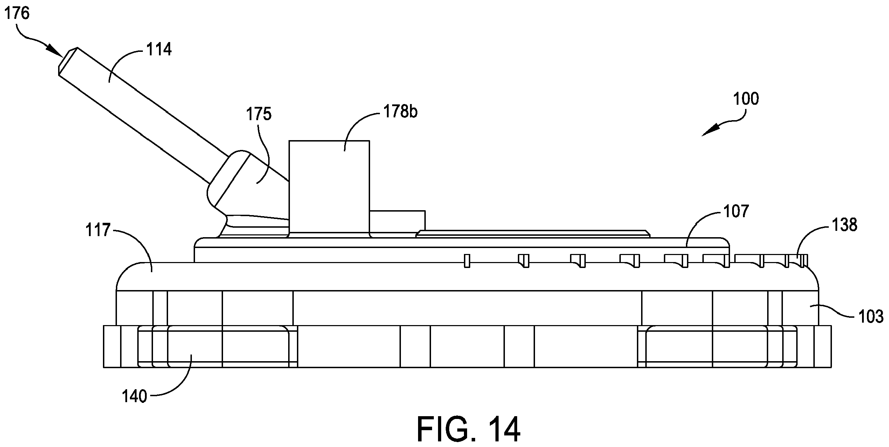

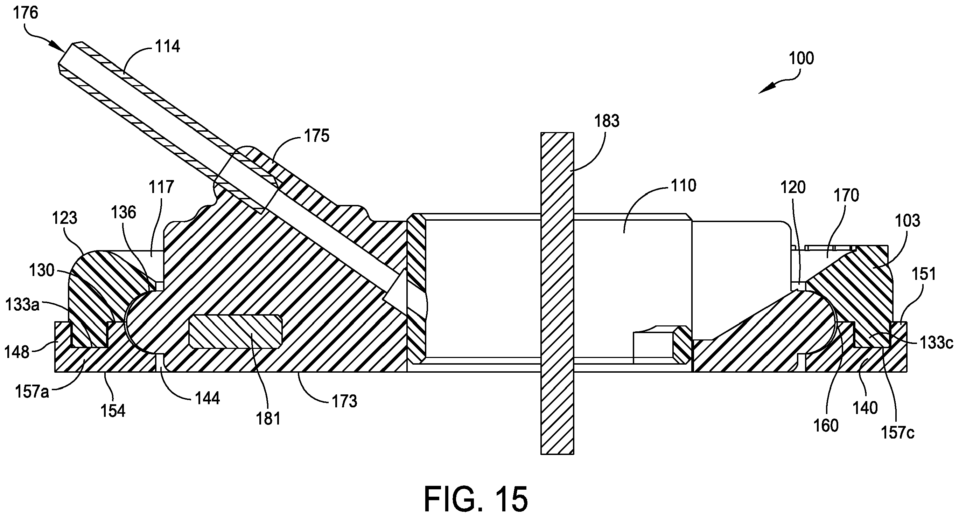

Referring now to FIGS. 10-15, a third surgical guide 100 includes a body 103, an insert 107, a guide aperture 110, and an irrigation port 114. Body 103 has a top portion 117 with an inner edge 120, an outer edge 123, a top surface 127, and a bottom surface 130. Top portion 117 defines at least one protrusion 132a-d on outer edge 123 of top portion 117. Bottom surface 130 defines one or more securing members 133a-d. Bottom surface 130 also defines a first ridge 136 on inner edge 120. In some embodiments, surgical guide 100 may also include a plurality of indicia 138 (i.e., ridges, lines, tick marks, etc.) disposed around top surface 127 of top portion 117. Body 103 also has a bottom portion 140 with an inner edge 144, an outer edge 148, a top surface 151, and a bottom surface 154. Bottom portion 140 defines at least one second protrusion 155a-d on outer edge 148 of bottom portion 140. Top surface 151 of bottom portion 140 defines one or more securing members 157a-d. Top surface 151 of bottom portion 140 also defines a second ridge 160 on inner edge 144 of bottom portion 140.

Securing members 133a-d on top portion 117 are configured to be received within securing members 157a-d on bottom portion 140, coupling top portion 117 and bottom portion 140 together and forming body 103. For example, securing members 133a-d and securing members 157a-d may press-fit together, forming body 103. Additionally, first protrusions 132a-d on top portion 117 are configured to align with second protrusions 155a-d to define at least one channel 163a-d. It should be understood that body 103 may define one or more channels 163a-d depending on the number of first protrusions 132a-d and second protrusions 155a-d. Channels 163a-d are configured to receive one or more fixation elements 167 to couple body 103 to one or more bones of a patient, e.g., k-wires, pins, screws or other similar structures. For example, one or more of fixation elements 167 can be inserted through channels 163a-d and inserted into the bone, fixing surgical guide 100 to the patient.

In some embodiments, the shape of body 103 may be circular, rectangular, square, or oblong. Additionally, the size and shape of body 103 and particularly bottom portion 140 may be formed so as to have patient-specific surface based on pre-operative fitting. The size and shape of body 103 and particularly bottom portion 140 may also be formed so as to be patient-specific based on imaging of the surgical site. While in some embodiments body 103 may be one piece, in other embodiments, body 103 may be formed of two pieces (i.e., top portion 117 and bottom portion 140), where the two pieces can be snapped or locked together. In some embodiments, surgical guide 100 may be formed from a medical-grade material that is capable of being 3D printed (e.g., additively manufactured), such as ABS (acrylonitrile butadiene styrene), PLA (polylactic acid), PETG (polyethylene terephthalate glycol), nylon, TPU (thermoplastic polyurethane), resin, and other suitable thermoplastics and thermosetting plastics. However, surgical guide 100 may be formed from other materials, including metals, ceramics, and other materials that are suitable for use in surgery as will be understood by one of ordinary skill in the art. In some embodiments, surgical guide 100 may be machined and/or formed using an additive manufacturing process, such as electron beam melting (EBM) or direct metal laser sintering (DMLS), to list only a few possibilities.

Surgical guide 100 also includes insert 107 having a top surface 170, a bottom surface 173, a receiving member 174, and a port 175. Insert 107 is disposed between first ridge 136 and second ridge 160. Insert 107 defines a receiving member 174 disposed through insert 107 between top surface 170 and bottom surface 173. Insert 107 also includes a port 175 disposed on top surface 170. Receiving member 174 is configured to receive guide aperture 110 therein. In some embodiments, insert 107 includes guide aperture 110, removing the need for receiving member 174. Port 175 is configured to receive irrigation port 114. Guide aperture 110 and irrigation port 114 define a canal 176 that extends from irrigation port 114 into guide aperture 110. In some embodiments, insert 107 may also include an indicator 177 (i.e., arrow, pointer, ledge, etc.) that may be aligned with guide aperture 110 so that as insert 107 is moved, indicator 177 points to one of the plurality of indicia 138 to indicate the angle of the guide aperture 110. Insert 107 also defines one or more holes 178a-b that communicate between top surface 170 and bottom surface 173 through insert 107. In some embodiments, insert 107 includes radiopaque markers 181 disposed within it. In some embodiments, the shape of insert 107 may be circular, rectangular, square, etc. in order to match the shape of body 103. Insert 107 is configured to move (i.e., rotate or translate) with respect to body 103. In some embodiments, insert 107 may be configured to rotate in 360 degrees. In some embodiments, body 103 and insert 107 are formed from different materials.

Guide aperture 110 is sized and configured to receive a surgical instrument 183, such as a drill, burr, etc. For example, guide aperture 110 may be a slot, such as a substantially rectangular slot, sized and configured to receive surgical instrument 183, such as a burr, to cut bone during a surgical procedure. In some embodiments, guide aperture 110 may be a hole, such as a circular hole, sized and configured to receive surgical instrument 183, such as a drill, to drill into the bone to a pre-determined depth. Guide aperture 110 may be made of metal to protect insert 107 from abrasion from surgical instrument 183. In other embodiments, guide aperture 110 may be formed of a polymer or other radiolucent material to be able to precisely see the location of the surgical instrument 183 through intraoperative imaging. Holes 178a-b defined in insert 107 are sized to receive fixation elements 167 that are used to lock insert 107 to place guide aperture 110 at a desired angle. For example, fixation elements 167 may include k-wire, pins, screws or other similar structures. In other embodiments, a locking device 187 may be used to fixate guide aperture 110 of insert 107 at a fixed angle rather than using one or more fixation elements 167 through holes 178a-b.

Irrigation port 114 provides access for a liquid (i.e., water, saline, etc.) to flow into the surgical site to cool the surgical site during a procedure. The liquid passing through irrigation port 114 may also be used to remove bone or tissue debris caused by the cutting or drilling. In some embodiments, irrigation port 114 may also be configured to receive a suction hose to provide suction to the surgical site in order to remove bone or tissue debris. Irrigation port 114 may also be configured to act as a handle that can be gripped or engaged with a tool, such as pliers, in order to facilitate removal of surgical guide 100 from the bone. In other embodiments, surgical guide 100 may include a handle 190 disposed within one of holes 178a-b or an alternate additional hole.

Surgical guide 100 may also include one or more radiopaque markers 181 disposed within insert 107. Radiopaque markers 181 may be used as reference markers to indicate a change in orientation of guide aperture 110 using a camera, fluoroscopy, x-ray, CT image. This allows a surgeon to intraoperatively move insert 107 to align guide aperture 110 at a desired angle. In some embodiments, radiopaque markers 181 may also be used in conjunction with a surgical navigation system, as discussed below, which may be configured to display the orientation of guide aperture 110 on a display. For example, radiopaque markers 181 may provide the surgical navigation system with markers that can be tracked as insert 107 is moved (i.e., rotated or translated).

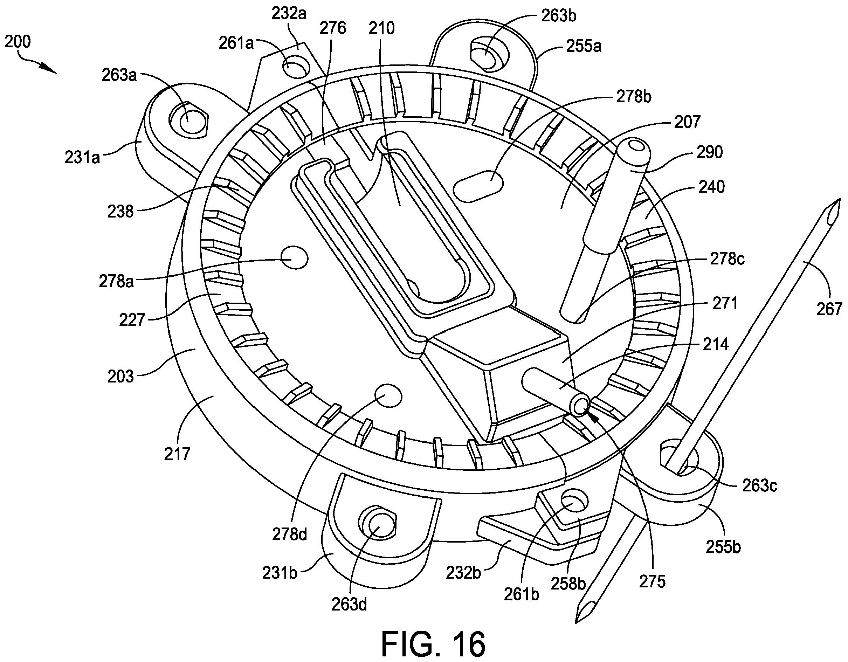

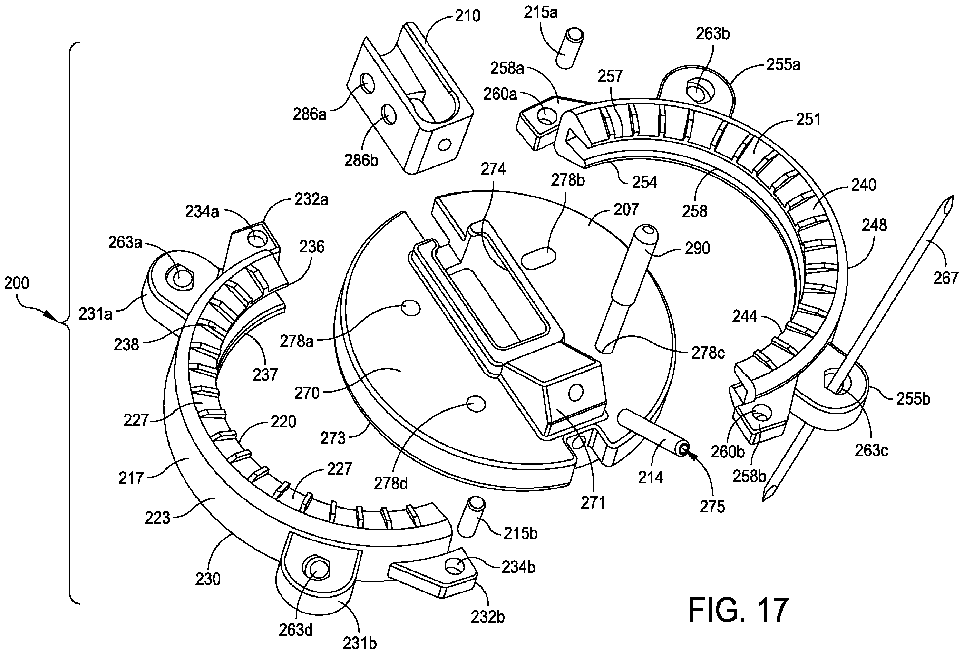

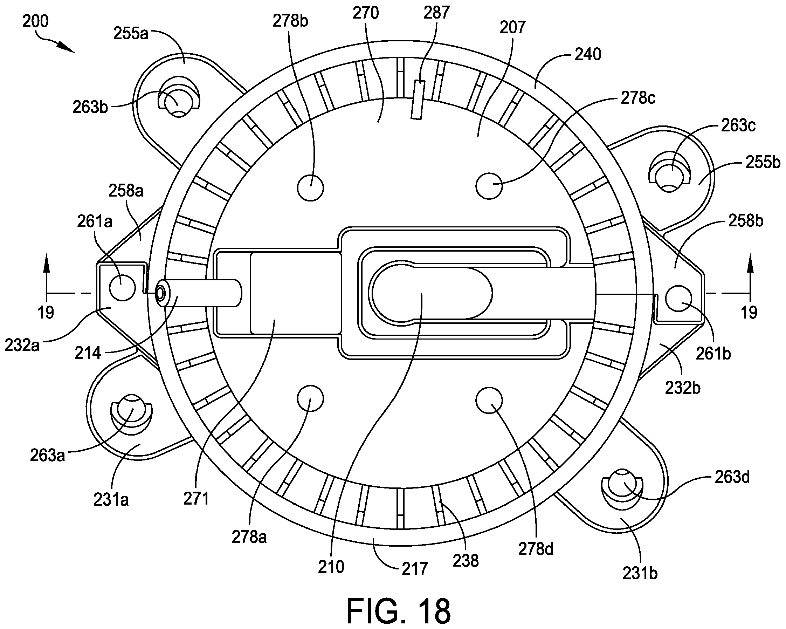

Referring now to FIGS. 16-19 a fourth surgical guide 200 includes a body 203, an insert 207, a guide aperture 210, an irrigation port 214, and two pins 215a-b. Body 203 has a left portion 217 with an inner edge 220, an outer edge 223, a top surface 227, and a bottom surface 230. Left portion 217 defines at least one first protrusion 231a-b on outer edge 223 of left portion 217. Left portion 217 of body 203 also has two securing members 232a-b disposed on outer edge 223 of left portion 217 that defines a first passage 234a-b through left portion 217. Inner edge 220 also defines a first ridge 236 and a second ridge 237. Body 203 also has a right portion 240 with an inner edge 244, an outer edge 248, a top surface 251, and a bottom surface 254. Right portion 240 defines at least one second protrusion 255a-b on outer edge 248 of right portion 240. Inner edge 244 of right portion 240 defines a first ridge 257 and a second ridge 258. Right portion 240 of body 203 has two securing members 258a-b disposed on outer edge 248 of right portion 240 that defines a second passage 260a-b through right portion 240. In some embodiments, surgical guide 200 may also include a plurality of indicia 238 (i.e., ridges, lines, tick marks, etc.) disposed around top surface 227 of left portion 217 and top surface 251 of right portion 240.

First passage 234a of securing member 232a and second passage 260a of securing member 258a are configured to align with one another so as to define a first void 261a in body 203. Pin 215a is sized to be received within first void 261a. Additionally, first passage 234b of securing member 232b and second passage 260b of securing member 258b are configured to align with one another so as to define a second void 261b in body 203. Pin 215b is sized to be received within second void 261b. Pin 225a through first void 261a and pin 225b through second void 261b couple left portion 217 to right portion 240.

Additionally, first protrusions 231a-b disposed on left portion 217 and second protrusions 255a-b disposed on right portion 240 define channels 263a-d. It should be understood that body 203 may define one or more channels 263a-d depending on the number of first protrusions 231a-b and second protrusions 255a-b. Channels 263a-d are configured to receive one or more fixation elements 267 to couple the body 203 to one or more bones of a patient, e.g., k-wires, pins, screws or other similar structures. For example, one or more of fixation elements 267 can be inserted through one or more channels 263a-d and inserted into the bone, fixing surgical guide 200 to the patient.

In some embodiments, the shape of body 203 may be circular, rectangular, square, or oblong. Additionally, the size and shape of body 203, and particularly bottom surface 230 of left portion 217 and bottom surface 254 of right portion 240, may be formed so as to have a patient-specific surface based on pre-operative fitting. The size and shape of body 203, and particularly bottom surface 230 of left portion 217 and bottom surface 254 of right portion 240, may also be formed so as to be patient-specific based on imaging of the surgical site. While in some embodiments body 203 may be one piece, in other embodiments, body 203 may be formed of two pieces (i.e., left portion 217 and right portion 240). In some embodiments, surgical guide 200 may be formed from a medical-grade material that is capable of being 3D printed (e.g., additively manufactured), such as ABS (acrylonitrile butadiene styrene), PLA (polylactic acid), PETG (polyethylene terephthalate glycol), nylon, TPU (thermoplastic polyurethane), resin, and other suitable thermoplastics and thermosetting plastics. However, surgical guide 200 may be formed from other materials, including metals, ceramics, and other materials that are suitable for use in surgery as will be understood by one of ordinary skill in the art. In some embodiments, surgical guide 200 may be machined and/or formed using an additive manufacturing process, such as electron beam melting (EBM) or direct metal laser sintering (DMLS), to list only a few possibilities.

Surgical guide 200 also includes insert 207 having a top surface 270, a port 271, a bottom surface 273, and a receiving member 274. Insert 207 is disposed between first ridge 236 and second ridge 237 of left portion 217 and first ridge 257 and second ridge 258 of right portion 240. Insert 207 defines receiving member 274 disposed through insert 207 between top surface 270 and bottom surface 273. Insert 207 also includes a port 271 disposed on top surface 270. Receiving member 274 is configured to receive guide aperture 210. In some embodiments, insert 207 includes guide aperture 210, removing the need for receiving member 274. Port 271 is configured to receive irrigation port 214. Guide aperture 210 and irrigation port 214 define a canal 275 that extends from irrigation port 214 into guide aperture 210. In some embodiments, insert 207 may also include an indicator 276 (i.e., arrow, pointer, ledge, etc.) that may be aligned with the guide aperture 210 so that as insert 207 is moved, indicator 276 points to one of a plurality of indicia 238 to indicate the angle of guide aperture 210. Insert 207 also defines one or more holes 278a-d that communicate from top surface 270 to bottom surface 273 through insert 207. In some embodiments, insert 207 includes radiopaque markers 281 disposed within insert 207. In some embodiments, the shape of insert 207 may be circular, rectangular, square, etc. in order to match the shape of body 203. Insert 207 is configured to move (i.e., rotate or translate) with respect to body 203. In some embodiments, insert 207 may be configured to rotate in 360 degrees. In some embodiments, body 203 and insert 207 are formed from different materials.

Guide aperture 210 is sized and configured to receive a surgical instrument 283, such as a drill, burr, etc. For example, guide aperture 210 may be a slot, such as a substantially rectangular slot, sized and configured to receive surgical instrument 283, such as a burr, to cut the bone during a surgical procedure. In some embodiments, guide aperture 210 may be an opening, such as a circular or oval opening, sized and configured to receive surgical instrument 283, such as a drill, to drill into the bone to a pre-determined depth. In some embodiments, guide aperture 210 includes one or more manufacturing features 286a-b. Guide aperture 210 may be made of metal to protect insert 207 from abrasive damage from surgical instrument 283. In other embodiments, guide aperture 210 may be made out of a plastic or other radiolucent material to be able to precisely see the location of the surgical instrument 283 through intraoperative imaging.

Holes 278a-d disposed in insert 207 are sized to receive fixation elements 267 that are used to lock insert 207 to place guide aperture 210 at a desired angle. For example, fixation elements 267 can include k-wire, pins, screws or other similar structures. In other embodiments, a locking device 287 may be used to fixate guide aperture 210 of insert 207 at a fixed angle rather than using one or more fixation elements 267 through holes 278a-b.

Irrigation port 214 allows liquid (i.e., water, saline, etc.) to flow into the surgical site to cool the surgical site during a procedure. The liquid passing through irrigation port 214 may also be used to remove bone or tissue debris caused by the cutting or drilling. For example, liquid through canal 275 and manufacturing features 286a-b may be used to remove bone or tissue debris from guide aperture 210. In some embodiments, irrigation port 214 may also be configured to receive a suction hose to provide suction to the surgical site in order to remove bone or tissue debris. Irrigation port 214 may also be configured to act as a handle that can be gripped or engaged with a tool, such as pliers, in order to facilitate removal of surgical guide 200 from the bone. In other embodiments, surgical guide 200 may include a handle 290 disposed within one of the holes 278a-d or from an alternate additional hole.

Surgical guide 200 may also include one or more radiopaque markers 281 disposed within insert 207. Radiopaque markers 281 may be used as reference markers to indicate a change in orientation of guide aperture 210 using a camera, fluoroscopy, x-ray, CT image. This allows a surgeon to intraoperatively move insert 207 to align guide aperture 210 at a desired angle. In some embodiments, radiopaque markers 281 may also be used in conjunction with a surgical navigation system, as discussed below, which may be configured to display the orientation of guide aperture 210 on a display. For example, radiopaque markers 281 may provide the surgical navigation system with markers that can be tracked as insert 207 is moved (i.e., rotated or translated). In some embodiments, pins 215a-b may also be used as markers, similar to radiopaque markers 281, that communicate the position of body 203 to the surgical navigation system.

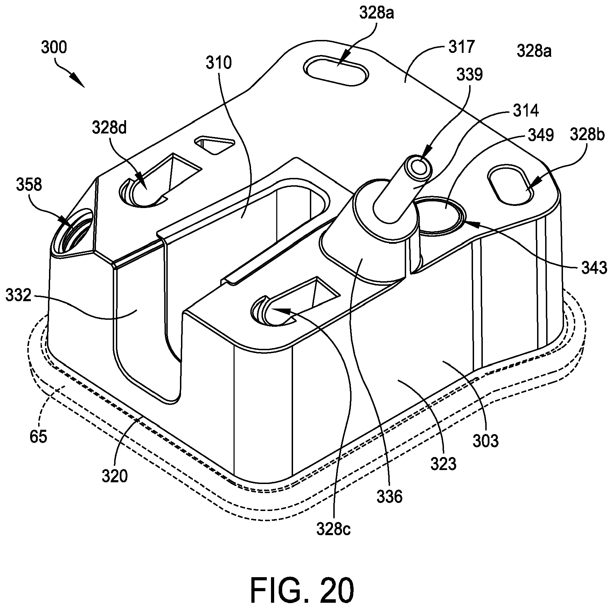

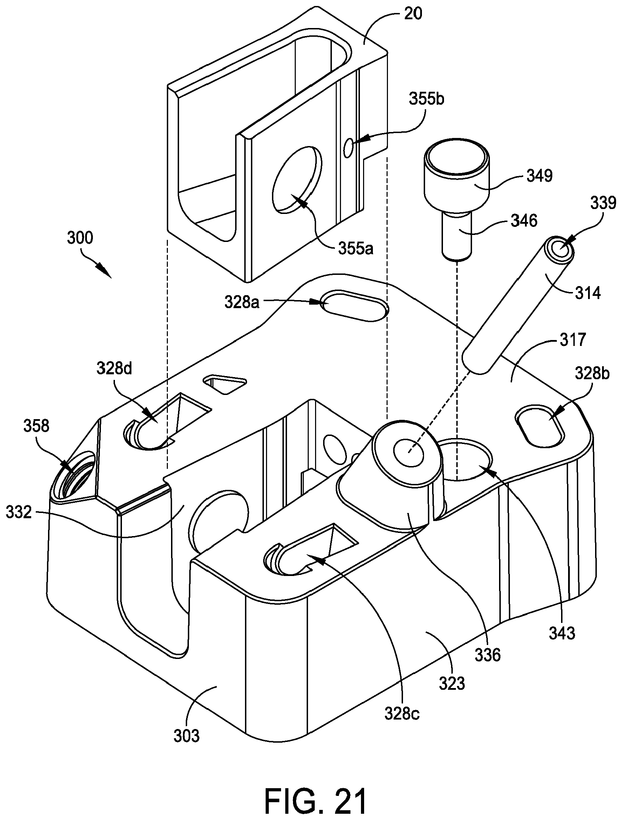

Referring now to FIGS. 20-23, a fifth surgical guide 300 includes a body 303, a guide aperture 310, and an irrigation port 314. Body 303 has a top surface 317, a bottom surface 320, a raised portion 323 extending between top surface 317 and bottom surface 320, and optionally a ring 65. Body 303 defines one or more channels 328a-d that are configured to receive one or more fixation elements, such as fixation element 267 illustrated in FIG. 16, to couple body 303 to one or more bones of a patient, e.g., k-wires, pins, screws or other similar structures. For example, one or more fixation elements can be inserted through one or more channels 328a-d and inserted into the bone, fixing surgical guide 300 to the patient. In some embodiments, one or more of the channels 328a-d are disposed at an angle such that fixation elements are inserted and disposed within channels 328a-d at some angle with reference to body 303. Body 303 also defines receiving surface 332 extending between top surface 317 and bottom surface 320. Body 303 also defines a port 336 disposed on top surface 317. Receiving surface 332 is configured to receive guide aperture 310. In some embodiments, body 303 includes guide aperture 310, removing the need for receiving surface 332. Port 336 is configured to receive irrigation port 314. Guide aperture 310 and irrigation port 314 define a canal 339 that extends from irrigation port 314 into guide aperture 310.

In some embodiments, the shape of body 303 may be substantially circular, rectangular, square, or oblong. In some embodiments, body 303 is part of a standalone surgical guide 300. In other embodiments, body 303 can replace one or more of the inserts (e.g., inserts 17, 107, and 207) described herein such that body 303 is free to rotate or translate. For example ring 65 of body 303 may be disposed within the ridges of surgical guide 10, 93, 100, or 200. Additionally, the size and shape of body 303, and particularly bottom surface 320 may be formed so as to have a patient-specific surface based on pre-operative fitting. The size and shape of body 303, and particularly bottom surface 320, may also be formed so as to be patient-specific based on imaging of the surgical site. In some embodiments, surgical guide 300 may be formed from a medical-grade material that is capable of being 3D printed (e.g., additively manufactured), such as ABS (acrylonitrile butadiene styrene), PLA (polylactic acid), PETG (polyethylene terephthalate glycol), nylon, TPU (thermoplastic polyurethane), resin, and other suitable thermoplastics and thermosetting plastics. However, surgical guide 300 may be formed from other materials, including metals, ceramics, and other materials that are suitable for use in surgery as will be understood by one of ordinary skill in the art. In some embodiments, surgical guide 300 may be machined and/or formed using an additive manufacturing process, such as electron beam melting (EBM) or direct metal laser sintering (DMLS), to list only a few possibilities.

In some embodiments, top surface 317 defines an aperture 343 that is sized and configured to receive a sight 346 and an alignment ring 349. Sight 346 and alignment ring 349 are used to reduce parallax error and verify that surgical guide 300 and a surgical instrument are aligned. For example, when surgical guide 300 and a surgical instrument are correctly aligned, a surgeon will see sight 346 in the middle of alignment ring 349. When surgical guide 300 and a surgical instrument are misaligned, sight 346 will no longer be centered in alignment ring 349 and may appear to fully or partially disappear from view. In some embodiments, raised portion 323 defines one or more holding features 351a-b. Holding feature 351a includes two shoulders 353a-b. Holding feature 351b includes two shoulders 353c-d. Each of the shoulders 353a-d define respective bumps 354a-d. The pair of shoulders 353a-b and 353c-d are sized and configured to receive a fixation element, such as fixation element 267 illustrated in FIG. 16. The pair of shoulders 353a-b and 353c-d are configured to flex within the respective holding feature 351a-b when a fixation element is inserted into holding feature 351a-b to ensure the fixation element is tight against the bumps 354a-d, holding the fixation element in place and storing energy against the shoulders 353a-d.

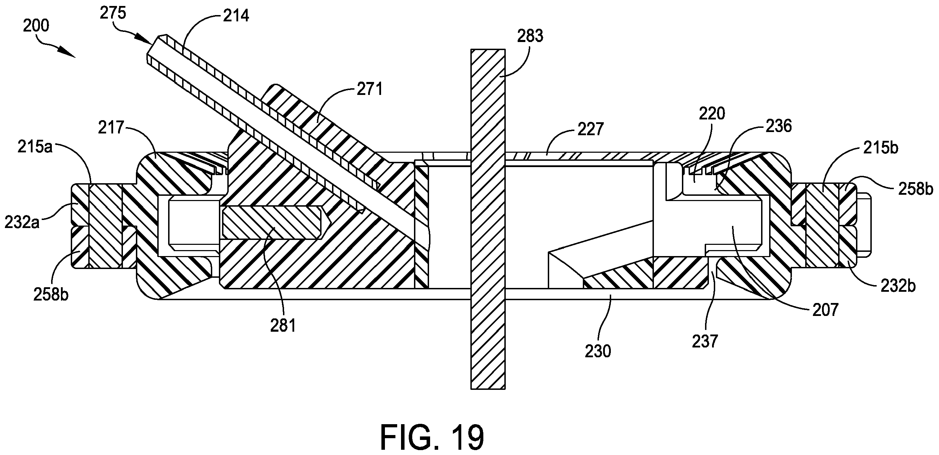

Guide aperture 310 is sized and configured to receive a surgical instrument, such as surgical instrument 283 illustrated in FIG. 19, which may be a drill, burr, etc. For example, guide aperture 310 may be a slot, such as a substantially wedge shape, sized and configured to receive a surgical instrument, such as a burr, to cut the bone during a surgical procedure or a drill, to drill into the bone to a pre-determined depth. In some embodiments, guide aperture 310 includes one or more manufacturing features 355a-b. Guide aperture 310 may be made of metal to protect body 303 from abrasive damage from a surgical instrument. In other embodiments, guide aperture 310 may be made out of a plastic or other radiolucent material to be able to precisely see the location of the surgical instrument through intraoperative imaging.

Irrigation port 314 allows liquid (i.e., water, saline, etc.) to flow into the surgical site to cool the surgical site during a procedure. The liquid passing through irrigation port 314 may also be used to remove bone or tissue debris caused by the cutting or drilling. For example, liquid through canal 339 and manufacturing features 355a-b may be used to remove bone or tissue debris from guide aperture 310. In some embodiments, irrigation port 314 may also be configured to receive a suction hose to provide suction to the surgical site in order to remove bone or tissue debris. Irrigation port 314 may also be configured to act as a handle that can be gripped or engaged with a tool, such as pliers, in order to facilitate removal of surgical guide 300 from the bone. In other embodiments, surgical guide 300 may include a handle, such as handle 290 illustrated in FIG. 16, disposed within one of the channels 328a-d or from an alternate additional hole 358.

Surgical guide 300 may also include one or more radiopaque markers, such as radiopaque marker 281 illustrated in FIG. 19, disposed within body 303. Radiopaque markers may be used as reference markers to indicate a change in orientation of guide aperture 310 using a camera, fluoroscopy, x-ray, CT image. This allows a surgeon to intraoperatively move body 303 to align guide aperture 310 at a desired angle. In some embodiments, radiopaque markers may also be used in conjunction with a surgical navigation system, as discussed below, which may be configured to display the orientation of guide aperture 310 on a display. For example, radiopaque markers may provide the surgical navigation system with markers that can be tracked as body 303 is moved (i.e., rotated or translated). In some embodiments, sight 346 may also be used as a marker, similar to radiopaque markers, that communicate the position of body 303 to the surgical navigation system.

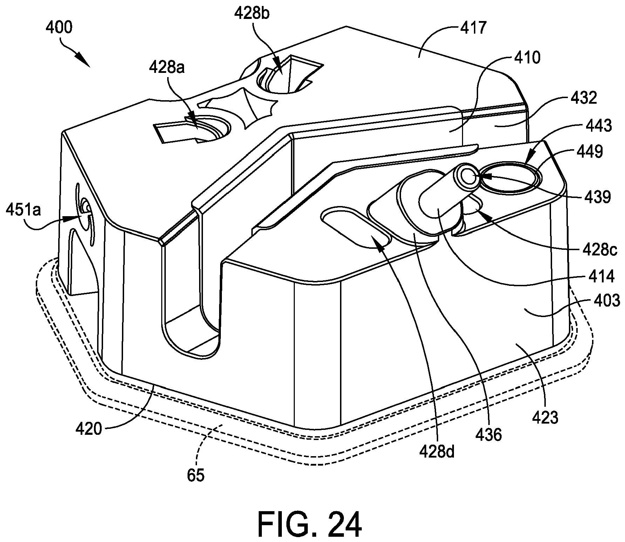

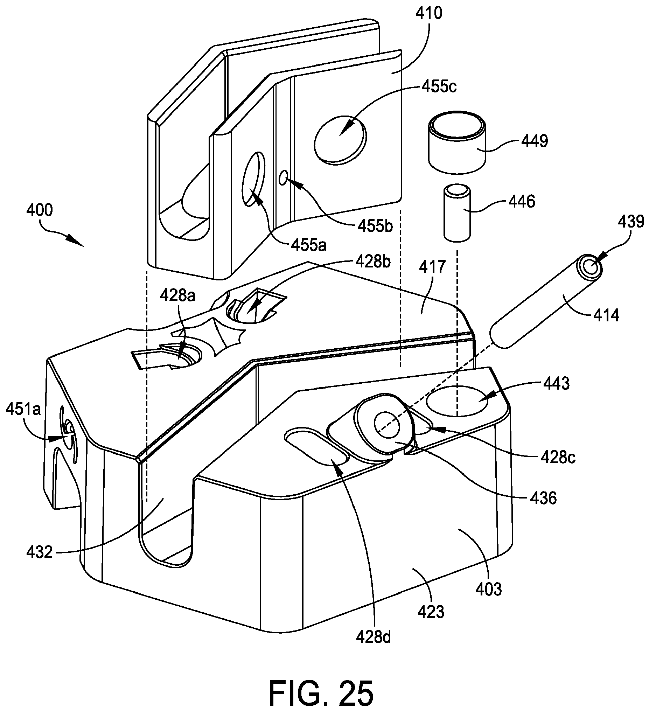

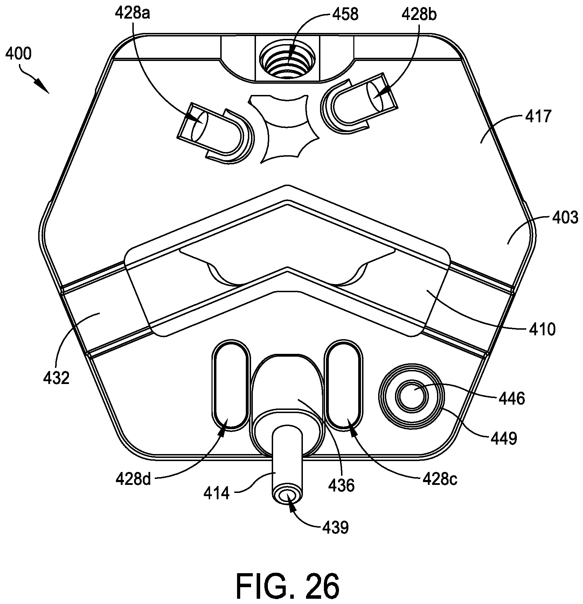

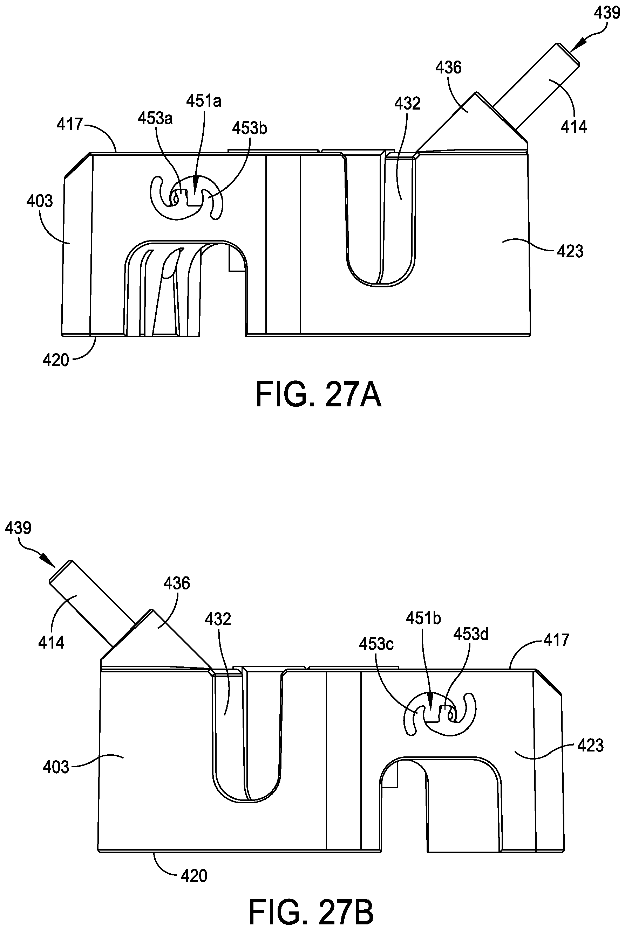

Referring now to FIGS. 24-27B, a sixth surgical guide 400 includes a body 403, a guide aperture 410, and an irrigation port 414. Body 403 has a top surface 417, a bottom surface 420, a raised portion 423 extending between top surface 417 and bottom surface 420, and optionally a ring 65.

Body 403 defines one or more channels 428a-d. Channels 428a-d are configured to receive one or more fixation elements, such as fixation element 267 illustrated in FIG. 16, to couple body 403 to one or more bones of a patient, e.g., k-wires, pins, screws or other similar structures. For example, one or more fixation elements can be inserted through one or more channels 428a-d and inserted into the bone, fixing surgical guide 400 to the patient. In some embodiments, one or more of the channels 428a-d are disposed at an angle such that fixation elements are inserted and disposed within channels 428a-d at some angle with reference to body 403. Body 403 also defines receiving surface 432 extending between top surface 417 and bottom surface 420. Body 403 also defines a port 436 disposed on top surface 417. Receiving surface 432 is configured to receive guide aperture 410. In some embodiments, body 403 includes guide aperture 410, removing the need for receiving surface 432. Port 436 is configured to receive irrigation port 414. Guide aperture 410 and irrigation port 414 define a canal 439 that extends from irrigation port 414 into guide aperture 410.

In some embodiments, the shape of body 403 may be substantially circular, rectangular, square, or oblong. In some embodiments, body 403 is part of a standalone surgical guide 400. In other embodiments, body 403 can replace one or more of the inserts (e.g., inserts 17, 107, and 207) described herein such that body 403 is free to rotate or translate. For example ring 65 of body 403 may be disposed within the ridges of surgical guide 10, 93, 100, or 200. Additionally, the size and shape of body 403, and particularly bottom surface 420 may be formed so as to have a patient-specific surface based on pre-operative fitting. The size and shape of body 403, and particularly bottom surface 420, may also be formed so as to be patient-specific based on imaging of the surgical site. In some embodiments, surgical guide 400 may be formed from a medical-grade material that is capable of being 3D printed (e.g., additively manufactured), such as ABS (acrylonitrile butadiene styrene), PLA (polylactic acid), PETG (polyethylene terephthalate glycol), nylon, TPU (thermoplastic polyurethane), resin, and other suitable thermoplastics and thermosetting plastics. However, surgical guide 400 may be formed from other materials, including metals, ceramics, and other materials that are suitable for use in surgery as will be understood by one of ordinary skill in the art. In some embodiments, surgical guide 400 may be machined and/or formed using an additive manufacturing process, such as electron beam melting (EBM) or direct metal laser sintering (DMLS), to list only a few possibilities.

In some embodiments, top surface 417 defines an aperture 443 that is sized and configured to receive a sight 446 and an alignment ring 449. Sight 446 and alignment ring 449 are used to reduce parallax error and verify that surgical guide 400 and a surgical instrument are aligned. For example, when surgical guide 400 and a surgical instrument are correctly aligned, a surgeon will see sight 446 in the middle of alignment ring 449. When surgical guide 400 and a surgical instrument are misaligned, sight 446 will no longer be centered in alignment ring 449 and may appear to fully or partially disappear from view.

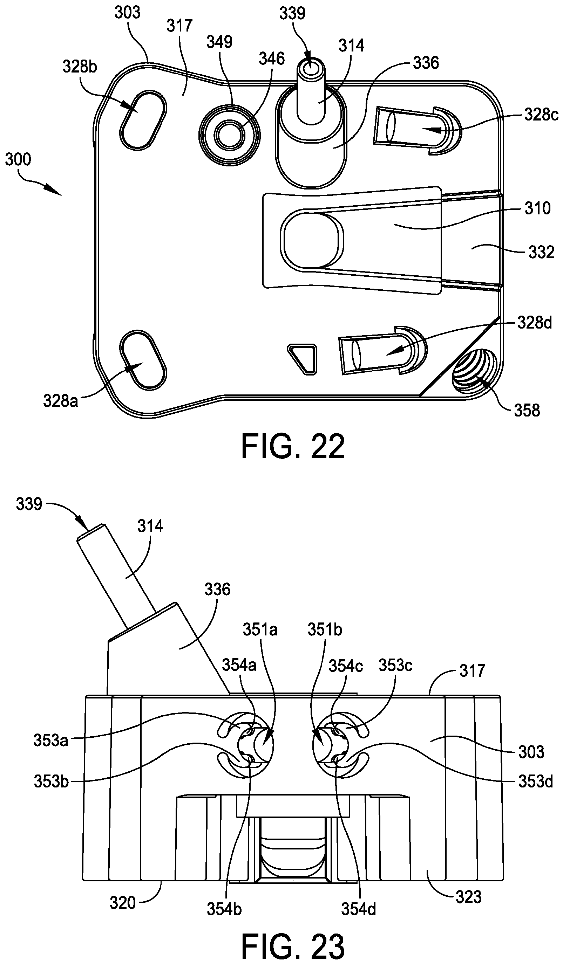

In some embodiments, raised portion 423 defines one or more holding features 451a-b. Holding feature 451a includes two shoulders 453a-b. Holding feature 451b includes two shoulders 453c-d. Each of the shoulders 453a-d define respective bumps, similar to bumps 354a-d illustrated in FIG. 23. The pair of shoulders 453a-b and 453c-d are sized and configured to receive a fixation element, such as fixation element 267 illustrated in FIG. 16. The pair of shoulders 453a-b and 453c-d are configured to flex within the respective holding feature 451a-b when a fixation element is inserted into holding feature 451a-b to ensure the fixation element is tight against the bumps, holding the fixation element in place and storing energy against the shoulders 453a-d.

Guide aperture 410 is sized and configured to receive a surgical instrument, such as surgical instrument 283 illustrated in FIG. 19, which may be a drill, burr, etc. For example, guide aperture 410 may be a slot, such as a substantially chevron shape, sized and configured to receive a surgical instrument, such as a burr, to cut the bone during a surgical procedure or a drill, to drill into the bone to a pre-determined depth. In some embodiments, guide aperture 410 includes one or more manufacturing features 455a-c. Guide aperture 410 may be made of metal to protect body 403 from abrasive damage from a surgical instrument. In other embodiments, guide aperture 410 may be made out of a plastic or other radiolucent material to be able to precisely see the location of the surgical instrument through intraoperative imaging.

Irrigation port 414 allows liquid (i.e., water, saline, etc.) to flow into the surgical site to cool the surgical site during a procedure. The liquid passing through irrigation port 414 may also be used to remove bone or tissue debris caused by the cutting or drilling. For example, liquid through canal 439 and manufacturing features 455a-c may be used to remove bone or tissue debris from guide aperture 410. In some embodiments, irrigation port 414 may also be configured to receive a suction hose to provide suction to the surgical site in order to remove bone or tissue debris. Irrigation port 414 may also be configured to act as a handle that can be gripped or engaged with a tool, such as pliers, in order to facilitate removal of surgical guide 400 from the bone. In other embodiments, surgical guide 400 may include a handle, such as handle 290 illustrated in FIG. 16, disposed within one of the channels 428a-d or from an alternate additional hole 458.

Surgical guide 400 may also include one or more radiopaque markers, such as radiopaque marker 281 illustrated in FIG. 19, disposed within body 403. Radiopaque markers may be used as reference markers to indicate a change in orientation of guide aperture 410 using a camera, fluoroscopy, x-ray, CT image. This allows a surgeon to intraoperatively move body 403 to align guide aperture 410 at a desired angle. In some embodiments, radiopaque markers may also be used in conjunction with a surgical navigation system, as discussed below, which may be configured to display the orientation of guide aperture 410 on a display. For example, radiopaque markers may provide the surgical navigation system with markers that can be tracked as body 403 is moved (i.e., rotated or translated). In some embodiments, sight 446 may also be used as a marker, similar to radiopaque markers, that communicate the position of body 403 to the surgical navigation system.

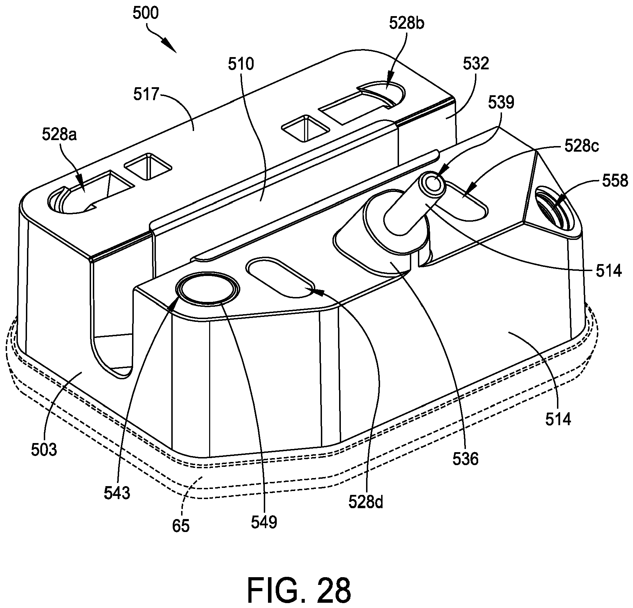

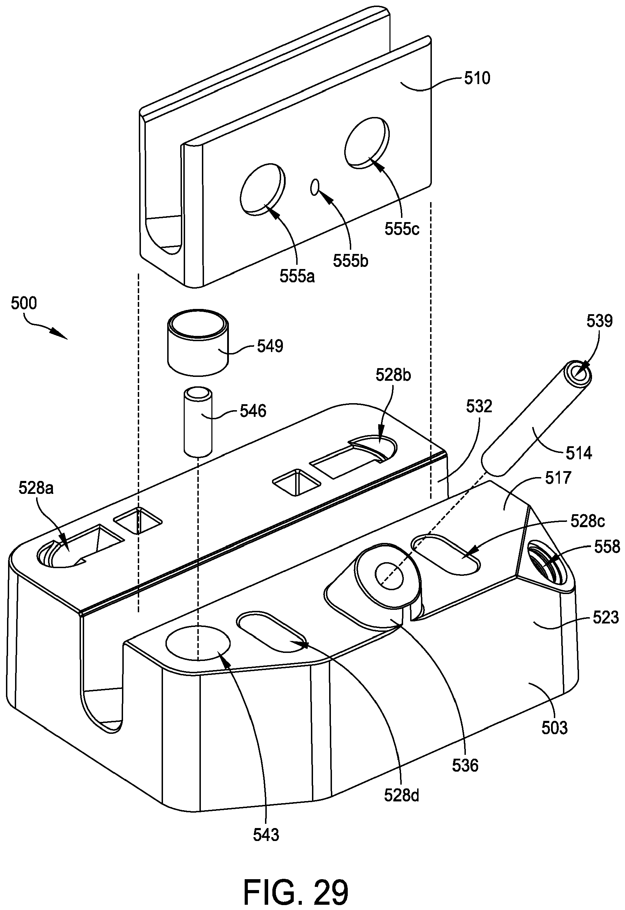

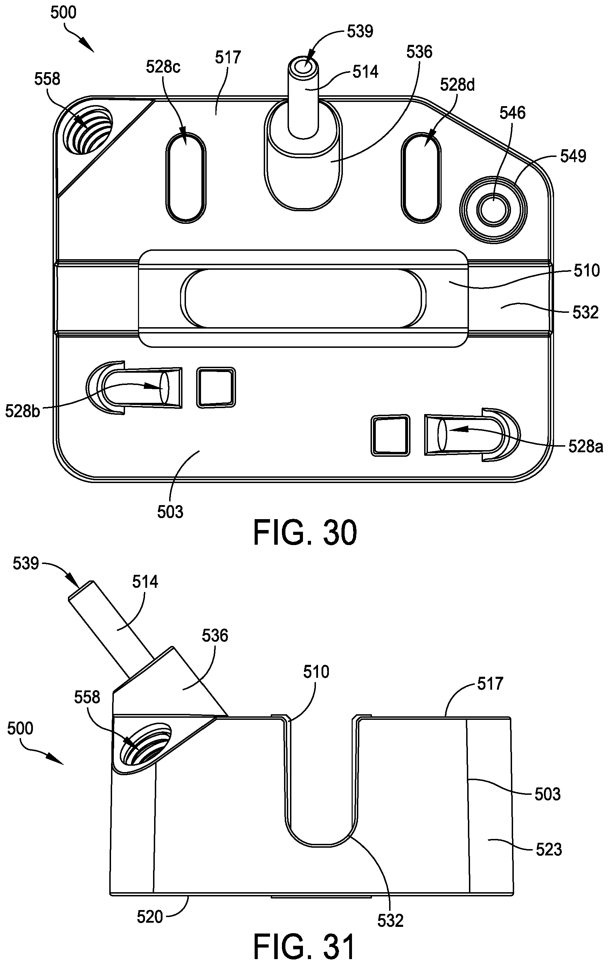

Referring now to FIGS. 28-31, a seventh surgical guide 500 includes a body 503, a guide aperture 510, and an irrigation port 514. Body 503 has a top surface 517, a bottom surface 520, a raised portion 523 extending between top surface 517 and bottom surface 520, and optionally a ring 65. Body 503 further defines one or more channels 528a-d. Channels 528a-d are configured to receive one or more fixation elements, such as fixation element 267 illustrated in FIG. 16, to couple body 503 to one or more bones of a patient, e.g., k-wires, pins, screws or other similar structures. For example, one or more fixation elements can be inserted through one or more channels 528a-d and inserted into the bone, fixing surgical guide 500 to the patient. In some embodiments, one or more of the channels 528a-d are disposed at an angle such that fixation elements are inserted and disposed within channels 528a-d at some angle with reference to body 503. Body 503 also defines receiving surface 532 extending between top surface 517 and bottom surface 520. Body 503 also defines a port 536 disposed on top surface 517. Receiving surface 532 is configured to receive guide aperture 510. In some embodiments, body 503 includes guide aperture 510, removing the need for receiving surface 532. Port 536 is configured to receive irrigation port 514. Guide aperture 510 and irrigation port 514 define a canal 539 that extends from irrigation port 514 into guide aperture 510.

In some embodiments, the shape of body 503 may be substantially circular, rectangular, square, or oblong. In some embodiments, body 503 is part of a standalone surgical guide 500. In other embodiments, body 503 can replace one or more of the inserts (e.g., inserts 17, 107, and 207) described herein such that body 503 is free to rotate or translate. For example ring 65 of body 503 may be disposed within the ridges of surgical guide 10, 93, 100, or 200. Additionally, the size and shape of body 503, and particularly bottom surface 520 may be formed so as to have a patient-specific surface based on pre-operative fitting. The size and shape of body 503, and particularly bottom surface 520, may also be formed so as to be patient-specific based on imaging of the surgical site. In some embodiments, surgical guide 500 may be formed from a medical-grade material that is capable of being 3D printed (e.g., additively manufactured), such as ABS (acrylonitrile butadiene styrene), PLA (polylactic acid), PETG (polyethylene terephthalate glycol), nylon, TPU (thermoplastic polyurethane), resin, and other suitable thermoplastics and thermosetting plastics. However, surgical guide 500 may be formed from other materials, including metals, ceramics, and other materials that are suitable for use in surgery as will be understood by one of ordinary skill in the art. In some embodiments, surgical guide 500 may be machined and/or formed using an additive manufacturing process, such as electron beam melting (EBM) or direct metal laser sintering (DMLS), to list only a few possibilities.

In some embodiments, top surface 517 defines an aperture 543 that is sized and configured to receive a sight 546 and an alignment ring 549. Sight 546 and alignment ring 549 are used to reduce parallax error and verify that surgical guide 500 and a surgical instrument are aligned. For example, when surgical guide 500 and a surgical instrument are correctly aligned, a surgeon will see sight 546 in the middle of alignment ring 549. When surgical guide 500 and a surgical instrument are misaligned, sight 546 will no longer be centered in alignment ring 549 and may appear to fully or partially disappear from view. Guide aperture 510 may be sized and configured to receive a surgical instrument, such as surgical instrument 283 illustrated in FIG. 19, which may be a drill, burr, etc. For example, guide aperture 510 may be a slot, such as a substantially transverse, sized and configured to receive a surgical instrument, such as a burr, to cut the bone during a surgical procedure or a drill, to drill into the bone to a pre-determined depth. In some embodiments, guide aperture 510 includes one or more manufacturing features 555a-c. Guide aperture 510 may be made of metal to protect body 503 from abrasive damage from a surgical instrument. In other embodiments, guide aperture 510 may be made out of a plastic or other radiolucent material to be able to precisely see the location of the surgical instrument through intraoperative imaging.

Irrigation port 514 allows liquid (i.e., water, saline, etc.) to flow into the surgical site to cool the surgical site during a procedure. The liquid passing through irrigation port 514 may also be used to remove bone or tissue debris caused by the cutting or drilling. For example, liquid through canal 539 and manufacturing features 555a-c may be used to remove bone or tissue debris from guide aperture 510. In some embodiments, irrigation port 514 may also be configured to receive a suction hose to provide suction to the surgical site in order to remove bone or tissue debris. Irrigation port 514 may also be configured to act as a handle that can be gripped or engaged with a tool, such as pliers, in order to facilitate removal of surgical guide 500 from the bone. In other embodiments, surgical guide 500 may include a handle, such as handle 290 illustrated in FIG. 16, disposed within one of the channels 528a-d or from an alternate additional hole 558.

Surgical guide 500 may also include one or more radiopaque markers, such as radiopaque marker 281 illustrated in FIG. 19, disposed within body 503. Radiopaque markers may be used as reference markers to indicate a change in orientation of guide aperture 510 using a camera, fluoroscopy, x-ray, CT image. This allows a surgeon to intraoperatively move body 503 to align guide aperture 510 at a desired angle. In some embodiments, radiopaque markers may also be used in conjunction with a surgical navigation system, as discussed below, which may be configured to display the orientation of guide aperture 510 on a display. For example, radiopaque markers may provide the surgical navigation system with markers that can be tracked as body 503 is moved (i.e., rotated or translated). In some embodiments, sight 546 may also be used as a marker, similar to radiopaque markers, that communicate the position of body 503 to the surgical navigation system.

Additionally, the systems and methods disclosed or suggested by this disclosure may use fluoro-based augmented reality to address problems, drawbacks, and issues with conventional surgical systems and methods. The fluoro-based augmented reality may be provided through the use of a combination of two-dimensional ("2D") to three-dimensional ("3D") estimation and/or registration of a surgical site, stereotaxy, and pattern recognition. 2D-to-3D estimation may include acquiring at least one first 2D image, which may be an x-ray image, and at least one second 2D image, which may also be an x-ray image, of a common site (e.g., the surgical site) where the first image is acquired in a first plane and the second image is acquired in a second plane that is disposed at an angle (e.g., an "imaging angle") relative to the first plane. In some embodiments, the imaging angle may be 60 degrees or more, for example. The two 2D images are used to estimate the physical features of the surgical site. For example, the two (or more) 2D images may be used to create a 3D estimation of one or more bones or other physical objects of the patient without the need to obtain one or more 3D images, such as one or more CT scans, of the patient.

2D-to-3D registration may include obtaining one or more 3D images, such as one or more CT scans and/or MRI images. The 3D images may be used to construct a 3D model of the surgical cite, including bone and/or cartilage, as will be understood by one of ordinary skill in the art. One or more 2D images may be obtained and one or more features in the 2D images may be registered to one or more features in the 3D model, as described in U.S. Patent No. 10,667,867, entitled "Methods of Pose Estimation of Three-Dimensional Bone Models in Surgical Planning a Total Ankle Replacement," the entirety of which is incorporated by reference herein.

In some embodiments, to facilitate the 2D-to-3D registration, a reference body may be used. The reference body may have a known shape and/or known dimensions to assist in the scaling of the images and the 3D registration. Further, one or more patterns may be provided on the reference body or other object to assist in the aligning the image planes. One example of such a reference body, which may take the form of a plurality of radiopaque spherical beads (or radiopaque markers 90, 181, 281), is described in U.S. Patent No. 10,105,168, entitled "Stereotactic Computer Assisted Surgery Based on Three-Dimensional Visualization," the entirety of which is incorporated by reference herein.

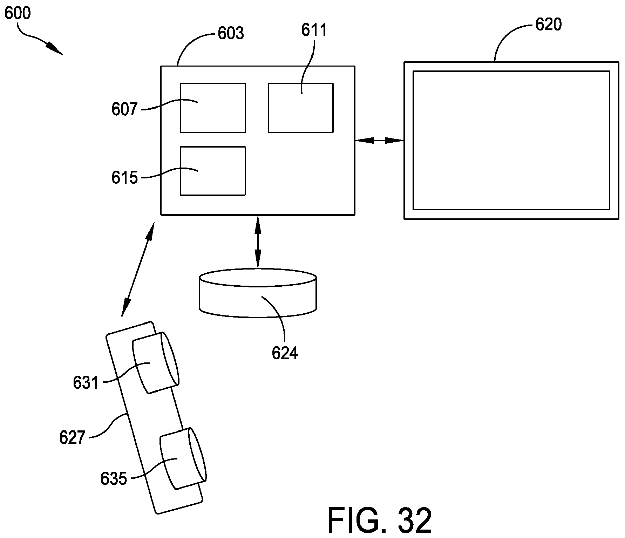

Referring to FIG. 32, a surgical navigation system 600 may include a computing device 603 with a processor 607, a memory 611, and an interface 615. Surgical navigation system 600 also includes a display 620, a data storage 624, and an imaging device 627. Surgical navigation system 600 may be used to guide the placement of surgical guide 10, 93, 100, 200, 300, 400, 500 during an osteotomy. However, it should be understood that surgical navigation system 600 may be used in connection with other surgical procedures, including revision procedures and surgical procedures involving other bones and joints.

Computing device 603 is coupled (e.g., by a wireless or a wired connection) to data storage 624, which may be a database, key-value data store, or other machine-readable storage device. In some embodiments, data storage 624 includes a database representative of a plurality bones with a plurality of sizes. The database of bones may include a patient-specific catalogue of the patient's bones based on pre-operative imaging. The database of bones may be based on an average size and shape of human bones determined by gender, age, weight, ethnicity, and/or medical history just to give a few examples. A person of ordinary skill in the art will appreciate other ways to determine bone size and shape.

Processor 607 obtains data from memory 611 and the data storage 624. Computing device 603 may be configured as a computer terminal located, for example, on a medical cart or may be a handheld tablet. Further, it should be understood that monitor or display 620 may be part of the computing device or may be separate from, but in communication with, computing device 603. One of ordinary skill in the art will understand that computing device 603 may take other forms.

Computing device 603 is also coupled to imaging device 627, which may be part of a surgical tracking system. Imaging device 627 may include one or more imaging units 631 and 635 (e.g., a C-arm or G-arm) that are configured to obtain images, such as fluoro images, in different planes. For example, if a C-arm with a single imaging unit 631 is used, then imaging device 627 may obtain a fluoroscopic image in a single plane. If a G-arm with multiple imaging units 631 and 635 is used, then imaging device 627 may obtain fluoroscopic images in multiple planes. The surgical navigation system 600, using imaging device 627, may be configured to track a reference body, such as one or more ribs, grooves, fiducial markers, radiopaque markers, or other objects that may be visible in a fluoroscopic image. For example, imaging device 627 may track radiopaque markers disposed within a surgical guide and transmit the position or movement of the radiopaque markers to computing device 603 to be displayed on display 620. Examples of the reference bodies may include the radiopaque markers 90, 181, 281 described above. Further disclosure for the surgical navigation system may be found in U.S. Provisional Application No. 63/371,095, entitled "Devices, Systems, and Methods for User Guidance in Surgical Navigation", the entirety of which is incorporated by reference herein.

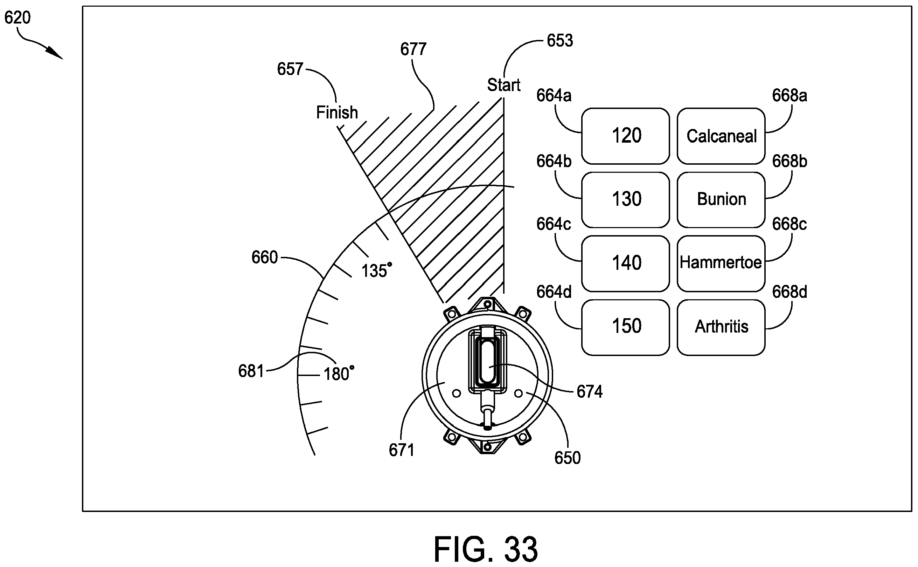

Referring to FIG. 33, a display 620 for displaying a surgical guide 650 includes a start indication 653, a finish indication 657, an angular tape 660, a plurality of angle softkeys 664a-d, and a plurality of surgery softkeys 668a-d. Computing device 603 of surgical navigation system 600 processes all adjustments of surgical guide 650 (e.g., surgical guide 10, 93, 100, 200, 300, 400, or 500) during a procedure and displays a graphical representation of surgical guide 650 having an insert 671 and guide aperture 674 on display 620. Start indication 653 and finish indication 657 may be referenced based on a reference marker, such as the one or more radiopaque markers 90, 181, or 281 discussed above, which may include pins 25a-b and 215a-b or sights 79, 346, 446, and 546. Start indication 653 and finish indication 657 may also be referenced to the bone of the patient, which could be based on pre-operative imaging or from the database of bones stored in data storage 624. In some embodiments, the display 620 includes a region 677 between start indication 653 and finish indication 657. Region 677 between the start indication 653 and finish indication 657 may be shaded or filled in with a different color to easily show how much more guide aperture 674 needs to move to reach the finish indication 657 while the insert 671 is rotated. In some embodiments, there may be a digital, aural, or physical (i.e., vibration) indication to stop moving the insert 671 once the guide aperture 674 has reached the finish indication 657. This may also include a change in color on the screen, a check mark popping up on the screen, etc. Angular tape 660 displays the degrees in defined values 681, such as every 2 degrees, 5 degrees, 10 degrees, 15 degrees, etc. as a quantitative representation of how much further the insert 671 needs to move to achieve the desired angle/location.

Display 620 also includes the option to quickly set the desired angle between start indication 653 and finish indication 657 by selection one of the plurality of angle softkeys 664a-d. The desired angle between the start indication 653 and the finish indication 657 may also be set manually or automatically based at least in part on a pre-operative plan, a post-operative plan, or an intraoperative decision. For example, a pre-operative plan may have determined that the angle between two bone cuts should be 70 degrees based on the size and shape of the patient's bone. Thus, 70 degrees is entered into surgical navigation system 600 and displayed on the display 620. However, the surgeon may determine during the operation for any number of reasons that the angle of the two bone cuts should be 65 degrees. Surgical navigation system 600 allows for a user to enter the new angle between the start indication 653 and finish indication 657 into the computing device 603 through interface 615, such as a keypad or touchscreen.

The display 620 may also include the option to select the type of surgery that is going to be performed with one of the plurality of surgery softkeys 668a-d. For example, surgery softkeys 668a-d may include options for a bunion procedure, calcaneal osteotomy, hammertoe, etc. The selection of one of the different surgeries with surgery softkeys 668a-d determines the shape of the bone cut displayed on the display 620 between start indication 653 and finish indication 657.

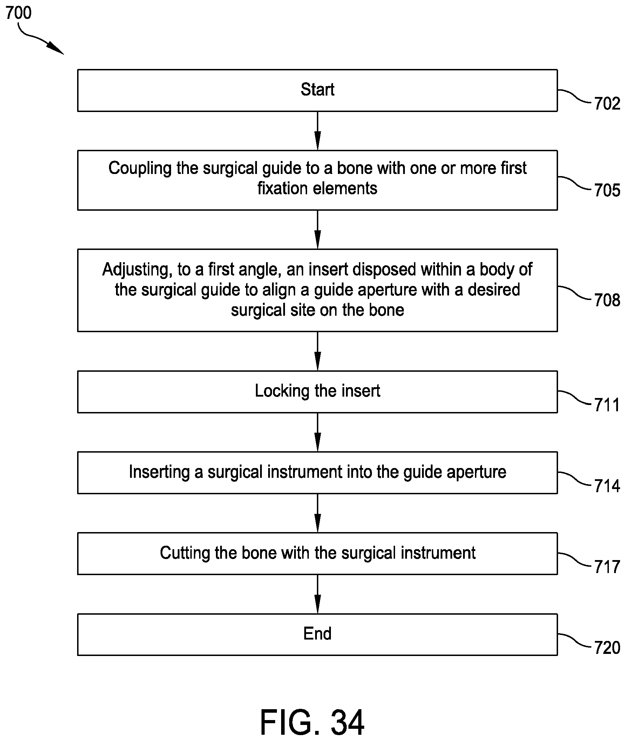

Referring now to FIG. 34, a method of using a surgical guide, such as surgical guide 10, 93, 100, 200, 300, 400, or 500, is disclosed. The following method 700 is described with reference to first surgical guide 10, but it shall be noted that method 700 may use other surgical guides, such as surgical guides 93, 100, 200, 300, 400, or 500. For example, method 700 of using a surgical guide for a surgical procedure, such as an osteotomy, starts at step 702. Method 700 continues to step 705, which includes coupling first surgical guide 10 to a bone by passing one or more fixation elements 64 through channels 63a-d and inserting the one or more fixation elements 64 into a bone. At step 708, the surgeon may adjust, to a first angle, insert 17 disposed within body 13 of surgical guide 10 to align guide aperture 20 with a pre-determined surgical site on the bone. Method 700 continues to step 711, which includes locking insert 17, such as by inserting one or more fixation elements 64 through holes 75a-b and into a bone or through the use of locking device 84 . The surgeon then inserts surgical instrument 81, such as a drill or burr, into the guide aperture 20 at step 714. Method 700 continues to step 717, where the surgeon makes a first cut of the bone with surgical instrument 81. Method 700 then ends at step 720. In some embodiments, method 700 also includes unlocking insert 17 by removing the one or more fixation elements 64 inserted through holes 75a-b or unlocking locking device 84, adjusting insert 17 to a second angle, and relocking the insert 17 at the second angle, such as by inserting one or more fixation elements 64 through holes 75a-b again and into a bone or through the use of locking device 84. The surgeon then makes a second cut of the bone with surgical instrument 81. In some embodiments, the first angle and the second angle are determined based at least in part on one of a pre-operative plan, a post-operative plan, or an intraoperative decision. In some embodiments, method 700 may further include aligning the surgical guide 10, facilitated by a sight and an alignment ring disposed within an aperture defined by the insert (e.g., sight 79 and alignment ring 80 disposed within aperture 78).

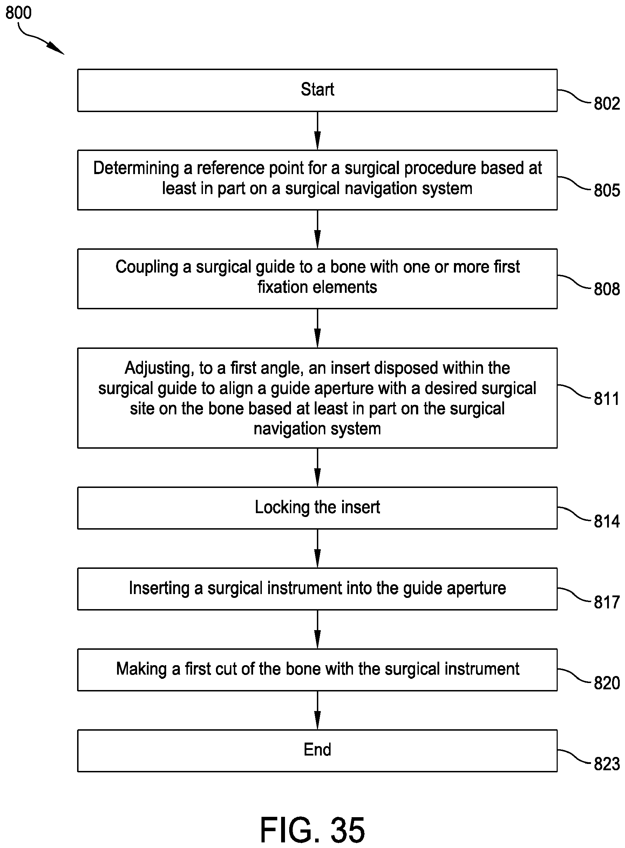

Referring now to FIG. 35, a method 800 for using a surgical system having surgical guide 10, 93, 100, 200, 300, 400, or 500 and a surgical navigation system 600 is disclosed. The following method 800 is described with reference to first surgical guide 10, but it shall be noted that method 800 may use other surgical guides, such as surgical guides 93, 100, 200, 300, 400, or 500. For example, method 800 of performing a surgical procedure, such as an osteotomy, starts at step 802. Method 800 continues to step 805, which includes determining a reference point for the osteotomy based at least in part on a surgical navigation system 600, reference markers 90 and bone. Step 808 includes coupling surgical guide 10 to a bone by passing one or more fixation elements 64 through channels 63a-d and into the bone. In step 811, the surgeon then adjusts, to a first angle, insert 17 disposed within body 13 of surgical guide 10 to align guide aperture 20 with a pre-determined surgical site on the bone based at least in part on surgical navigation system 600. In step 814, the surgeon locks insert 17, such as by inserting one or more fixation elements 64 through holes 75a-b and into the bone or through the use of locking device 84. In step 817, the surgeon then inserts surgical instrument 81 into the guide aperture 20. In step 820, the surgeon makes a first cut of the bone with surgical instrument 81. Method 800 ends at step 823.

In some embodiments, the surgeon then unlocks insert 17, adjusts insert 17 to a second angle determined based at least in part by the surgical navigation system 600, and re-locks insert 17 at the second angle. An imaging device 627 may be configured to display an adjustment of insert 17 on display 620 as insert 17 is moved. In some embodiments, display 620 is configured to display at least one of a pre-operative plan, a post-operative plan, and an intraoperative adjustment of guide aperture 20 based at least in part on a desired shape of the procedure. The surgeon then makes a second cut of the bone with surgical instrument 81. In some embodiments, the first angle and the second angle are determined based at least in part on at least one of a pre-operative plan, a post-operative plan, or intraoperative decision. The determination above may be based at least in part on a database of bones in data storage 624 configured to store the size and shapes of bones. In some embodiments, the method 800 may further include aligning the surgical guide 10, facilitated by a sight and an alignment ring disposed within an aperture defined by the insert (e.g., sight 79 and alignment ring 80 disposed within aperture 78).

### Features of the Inventions

In some embodiments, a surgical guide may include a body having a first portion with a first ridge and a second ridge, and a second portion with a first ridge and a second ridge. The surgical guide may also include an insert disposed between the first ridge and the second ridge of the first portion and the first ridge and second ridge of the second portion. The insert may be configured to rotate relative to the body. The surgical guide may also include a guide aperture disposed within the insert that is sized to receive a surgical instrument.

In some embodiments, the body may include a plurality of first protrusions and a plurality of second protrusions that define a plurality of channels configured to allow one or more fixation elements to pass through the body into a bone.

In some embodiments, the one or more fixation elements may be k-wires.

In some embodiments, the surgical guide may include an irrigation port configured to provide irrigation to a surgical site.

In some embodiments, the insert may include one or more holes disposed through the insert configured to allow one or more fixation elements to pass through the insert and into a bone.

In some embodiments, the insert may be locked with a locking device.

In some embodiments, the surgical guide may include a plurality of indicia configured to indicate a change in orientation of the guide aperture as the insert is rotated.

In some embodiments, the insert may include an indicator disposed on the insert configured to point to one of the plurality of the indicia.

In some embodiments, the insert may be rotatable in 360 degrees with respect to the body.

In some embodiments, the guide aperture may be a slot configured to receive the surgical instrument.

In some embodiments, the guide aperture may be an opening configured to receive the surgical instrument.

In some embodiments, the guide aperture may be a metal material.

In some embodiments, the guide aperture may be a radiolucent material.

In some embodiments, the insert may include a plurality of radiopaque markers disposed within the insert.

In some embodiments, the first portion and the second portion may be two separate parts that are coupled together with two pins.

In some embodiments, the body may be generally circular.

In some embodiments, the insert may be generally circular.

In some embodiments, the surgical guide may have a patient-specific surface.

In some embodiments, the patient-specific surface of the surgical guide may be based at least in part on pre-operative imaging of a bone.

In some embodiments, the guide aperture may be substantially rectangular, transverse, a wedge shape, or a chevron shape.

In some embodiments, a handle may be configured to remove the surgical guide from a bone.

In some embodiments, the insert may define one or more holding features configured to receive a fixation element.

In some embodiments, the insert may define an aperture that is sized and configured to receive a sight and an alignment ring.

In some embodiments, a surgical system may include a surgical guide. The surgical guide may include a body having a first portion with a first ridge and a second ridge, and a second portion with a first ridge and a second ridge. The surgical guide may also include an insert disposed between the first ridge and the second ridge of the first portion and the first ridge and second ridge of the second portion. The insert may be configured to rotate relative to the body. The surgical guide may also include a guide aperture disposed within the insert that is sized to receive a surgical instrument. The surgical system may also include a surgical navigation system having a computing device with a processor and a display configured to display intraoperative adjustments of the guide aperture.

In some embodiments, the surgical guide may include a plurality of first protrusions and a plurality of second protrusions that define a plurality of channels so as to allow one or more fixation elements to pass through the body into a bone.

In some embodiments, the one or more fixation elements may be k-wires.

In some embodiments, the surgical guide may include an irrigation port configured to provide irrigation to a surgical site.

In some embodiments, the insert may include one or more holes disposed through the insert configured to allow one or more fixation elements to pass through the insert and into a bone.

In some embodiments, the insert may be locked with a locking device.

In some embodiments, the surgical guide may include a plurality of indicia configured to indicate a change in orientation of the guide aperture as the insert is rotated.

In some embodiments, the insert may include an indicator disposed on the insert configured to point to one of the plurality of indicia.

In some embodiments, the insert may be rotatable in 360 degrees with respect to the body.

In some embodiments, the guide aperture may be a slot configured to receive the surgical instrument.

In some embodiments, the guide aperture may be an opening configured to receive the surgical instrument.

In some embodiments, the guide aperture may be a metal material.

In some embodiments, the guide aperture may be a radiolucent material.

In some embodiments, the insert may include a plurality of radiopaque markers disposed within the insert.

In some embodiments, the first portion and the second portion may be two separate parts that are coupled together with two pins.

In some embodiments, the body may be generally circular.

In some embodiments, the insert may be generally circular.

In some embodiments, the surgical guide may have a patient-specific surface.

In some embodiments, the patient-specific surface of the surgical guide may be based at least in part on pre-operative imaging of a bone.

In some embodiments, the guide aperture may be substantially rectangular, transverse, a wedge shape, or a chevron shape.

In some embodiments, the surgical guide may include a handle configured to remove the surgical guide from a bone.

In some embodiments, the surgical navigation system may include an imaging device communicatively coupled to the computing device configured to track a change in position of the insert and transmit the position of the insert to the computing device to be displayed on the display.

In some embodiments, the imaging device may be configured to track the position of the insert by tracking a movement of a plurality of radiopaque markers disposed within the insert as the insert is rotated.

In some embodiments, the surgical navigation system may be referenced based on an initial placement of the surgical guide on a bone with a pre-determined size.

In some embodiments, the insert may define one or more holding features configured to receive a fixation element.

In some embodiments, the insert may define an aperture that is sized and configured to receive a sight and an alignment ring.

In some embodiments, a method of using a surgical guide may include coupling the surgical guide to a bone with one or more first fixation elements. The method may also include adjusting, to a first angle, an insert disposed within a body of the surgical guide to align a guide aperture with a desired surgical site on the bone. The method may also include locking the insert. The method may also include inserting a surgical instrument into the guide aperture. The method may also include making a first cut of the bone with the surgical instrument.

In some embodiments, the method may include unlocking the insert, adjusting the insert to a second angle, and re-locking the insert at the second angle.

In some embodiments, the method may include making a second cut of the bone with the surgical instrument.

In some embodiments, the first angle and the second angle are referenced to the bone and are determined based at least in part on at least one of a pre-operative plan, a post-operative plan, or intraoperative decision.

In some embodiments, the method may further include aligning the surgical guide, facilitated by a sight and an alignment ring disposed within an aperture defined by the insert.

In some embodiments, a method of using a surgical system includes determining a reference point for a surgical procedure based at least in part on a surgical navigation system. The method may also include coupling a surgical guide to a bone with one or more first fixation elements. The method may also include adjusting, to a first angle, an insert disposed within the surgical guide to align a guide aperture with a desired surgical site on the bone based at least in part on the surgical navigation system. The method may also include locking the insert. The method may also include inserting a surgical instrument into the guide aperture. The method may also include making a first cut of the bone with the surgical instrument.

In some embodiments, the method may also include unlocking the insert, adjusting the insert to a second angle determined based at least in part by the surgical navigation system, and re-locking the insert at the second angle.

In some embodiments, the method may also include making a second cut of the bone with the surgical instrument.

In some embodiments, the first angle and the second angle are referenced to the bone and determined based at least in part on at least one of a pre-operative plan, a post-operative plan, or intraoperative decision.

In some embodiments, the determining step is based at least in part on a database of bones.

In some embodiments, the method may also include displaying the adjustment of the insert on a display.

In some embodiments, a display is configured to display at least one of a pre-operative plan, a post-operative plan, and an intraoperative adjustment of the guide aperture based at least in part on a desired shape of a procedure.

In some embodiments, the method may further include aligning the surgical guide, facilitated by a sight and an alignment ring disposed within an aperture defined by the insert.

In some embodiments, a surgical guide may include a body having a first portion with a first ridge and a second ridge, and a second portion with a first ridge and a second ridge. The surgical guide may also include an insert having a raised portion. The insert may be disposed between the first ridge and the second ridge of the first portion and the first ridge and second ridge of the second portion. The insert may be configured to rotate relative to the body. The surgical guide may also include a guide aperture disposed within the insert that is sized to receive a surgical instrument.

In some embodiments, the body may include a plurality of first protrusions and a plurality of second protrusions that define a plurality of channels configured to allow one or more fixation elements to pass through the body into a bone.

In some embodiments, the one or more fixation elements may be k-wires.

In some embodiments, the surgical guide may include an irrigation port configured to provide irrigation to a surgical site.

In some embodiments, the insert may include one or more holes disposed through the insert configured to allow one or more fixation elements to pass through the insert and into a bone.

In some embodiments, the insert may be locked with a locking device.

In some embodiments, surgical guide may include a plurality of indicia configured to indicate a change in orientation of the guide aperture as the insert is rotated.

In some embodiments, the insert may include an indicator disposed on the insert configured to point to one of the plurality of the indicia.

In some embodiments, the insert may be rotatable in 360 degrees with respect to the body.

In some embodiments, the guide aperture may be a slot configured to receive the surgical instrument.

In some embodiments, the guide aperture may be an opening configured to receive the surgical instrument.

In some embodiments, the guide aperture may be a metal material.

In some embodiments, the guide aperture may be a radiolucent material.

In some embodiments, the insert may include a plurality of radiopaque markers disposed within the insert.

In some embodiments, the first portion and the second portion are two separate parts that are coupled together with two pins.

In some embodiments, the body may be generally circular.

In some embodiments, the insert may be generally circular.

In some embodiments, the surgical guide may have a patient-specific surface.

In some embodiments, the patient-specific surface of the surgical guide may be based at least in part on pre-operative imaging of a bone.

In some embodiments, the guide aperture may be substantially rectangular, transverse, a wedge shape, or a chevron shape.

In some embodiments, further comprising a handle configured to remove the surgical guide from a bone.

In some embodiments, the insert may define one or more holding features configured to receive a fixation element.

In some embodiments, the insert may define an aperture that is sized and configured to receive a sight and an alignment ring.

In some embodiments, a surgical guide may include a body having a raised portion. The body may define one or more holding features configured to receive a fixation element. The body may also include an aperture that is sized and configured to receive a sight and an alignment ring. The surgical guide may also include a guide aperture disposed within the body that is sized to receive a surgical instrument. The surgical guide may also include an irrigation port configured to provide irrigation to a surgical site.

In some embodiments, the body may include one or more channels disposed through the body configured to allow one or more fixation elements to pass through the insert and into a bone.

In some embodiments, the body may include a ring to facilitate rotation of the body with respect to a second surgical guide.

In some embodiments, the guide aperture may be a slot configured to receive the surgical instrument.

In some embodiments, the guide aperture may be an opening configured to receive the surgical instrument.

In some embodiments, the guide aperture may be a metal material.

In some embodiments, the guide aperture may be a radiolucent material.

In some embodiments, the body may include a plurality of radiopaque markers disposed within the insert.

In some embodiments, the body may be substantially rectangular.

In some embodiments, the body may be substantially oblong.

In some embodiments, the surgical guide may have a patient-specific surface.

In some embodiments, the patient-specific surface of the surgical guide may be based at least in part on pre-operative imaging of a bone.

In some embodiments, the guide aperture may be substantially transverse, a wedge shape, or a chevron shape.

In some embodiments, further comprising a handle configured to remove the surgical guide from a bone.

Although the systems, guides, kits, and methods have been described in terms of exemplary embodiments, they are not limited thereto. Rather, the appended claims should be construed broadly, to include other variants and embodiments of the systems, guides, kits, and methods, which may be made by those skilled in the art without departing from the scope and range of equivalents.

Further disclosed herein is the subject-matter of the following clauses:
1. A surgical guide, comprising:
   a body having a first portion with a first ridge and a second ridge, and a second portion with a first ridge and a second ridge;
   an insert disposed between the first ridge and the second ridge of the first portion and the first ridge and second ridge of the second portion, wherein the insert is configured to rotate relative to the body; and
   a guide aperture disposed within the insert that is sized to receive a surgical instrument.
2. The surgical guide of clause 1, wherein the body includes a plurality of first protrusions and a plurality of second protrusions that define a plurality of channels configured to allow one or more fixation elements to pass through the body into a bone.
3. The surgical guide of clause 2, wherein the one or more fixation elements are k-wires.
4. The surgical guide of clause 1 or of any of the preceding clauses, wherein the surgical guide further comprises an irrigation port configured to provide irrigation to a surgical site.
5. The surgical guide of clause 1 or of any of the preceding clauses, wherein the insert further comprises one or more holes disposed through the insert configured to allow one or more fixation elements to pass through the insert and into a bone.
6. The surgical guide of clause 1 or of any of the preceding clauses, wherein the insert is locked with a locking device.
7. The surgical guide of clause 1 or of any of the preceding clauses, the surgical guide further comprises a plurality of indicia configured to indicate a change in orientation of the guide aperture as the insert is rotated.
8. The surgical guide of clause 7, wherein the insert further comprises an indicator disposed on the insert configured to point to one of the plurality of the indicia.
9. The surgical guide of clause 1 or of any of the preceding clauses, wherein the insert is rotatable in 360 degrees with respect to the body.
10. The surgical guide of clause 1 or of any of the preceding clauses, wherein the guide aperture is a slot configured to receive the surgical instrument.
11. The surgical guide of clause 1 or of any of the preceding clauses, wherein the guide aperture is an opening configured to receive the surgical instrument.
12. The surgical guide of clause 1 or of any of the preceding clauses, wherein the guide aperture is a metal material.
13. The surgical guide of clause 1 or of any of the preceding clauses, wherein the guide aperture is a radiolucent material.
14. The surgical guide of clause 1 or of any of the preceding clauses, wherein the insert further comprises a plurality of radiopaque markers disposed within the insert.
15. The surgical guide of clause 1 or of any of the preceding clauses, wherein the first portion and the second portion are two separate parts that are coupled together with two pins.
16. The surgical guide of clause 1 or of any of the preceding clauses, wherein the body is generally circular.
17. The surgical guide of clause 16, wherein the insert is generally circular.
18. The surgical guide of clause 1 or of any of the preceding clauses, wherein the surgical guide has a patient-specific surface.
19. The surgical guide of clause 18, wherein the patient-specific surface of the surgical guide is based at least in part on pre-operative imaging of a bone.
20. The surgical guide of clause 1 or of any of the preceding clauses, wherein the guide aperture is substantially rectangular, transverse, a wedge shape, or a chevron shape.
21. The surgical guide of clause 1 or of any of the preceding clauses, further comprising a handle configured to remove the surgical guide from a bone.
22. The surgical guide of clause 1 or of any of the preceding clauses, wherein the insert defines one or more holding features configured to receive a fixation element.
23. The surgical guide of clause 1 or of any of the preceding clauses, wherein the insert defines an aperture that is sized and configured to receive a sight and an alignment ring.
24. A surgical system comprising:
   a surgical guide, comprising:
      a body having a first portion with a first ridge and a second ridge, and a second portion with a first ridge and a second ridge;
      an insert disposed between the first ridge and the second ridge of the first portion and the first ridge and second ridge of the second portion, wherein the insert is configured to rotate relative to the body; and
      a guide aperture disposed within the insert that is sized to receive a surgical instrument; and
   a surgical navigation system having a computing device with a processor and a display configured to display intraoperative adjustments of the guide aperture.
25. The surgical system of clause 24, wherein the surgical guide includes a plurality of first protrusions and a plurality of second protrusions that define a plurality of channels so as to allow one or more fixation elements to pass through the body into a bone.
26. The surgical system of clause 25, wherein the one or more fixation elements are k-wires.
27. The surgical system of clause 24 or of any of clauses 24-26, wherein the surgical guide further comprises an irrigation port configured to provide irrigation to a surgical site.
28. The surgical system of clause 24 or of any of clauses 24-27, wherein the insert further comprises one or more holes disposed through the insert configured to allow one or more fixation elements to pass through the insert and into a bone.
29. The surgical system of clause 24 or of any of clauses 24-28, wherein the insert is locked with a locking device.
30. The surgical system of clause 24 or of any of clauses 24-29, wherein the surgical guide further comprises a plurality of indicia configured to indicate a change in orientation of the guide aperture as the insert is rotated.
31. The surgical system of clause 30, wherein the insert further comprises an indicator disposed on the insert configured to point to one of the plurality of indicia.
32. The surgical system of clause 24 or of any of clauses 24-31, wherein the insert is rotatable in 360 degrees with respect to the body.
33. The surgical system of clause 24 or of any of clauses 24-32, wherein the guide aperture is a slot configured to receive the surgical instrument.
34. The surgical system of clause 24 or of any of clauses 24-33, wherein the guide aperture is an opening configured to receive the surgical instrument.
35. The surgical system of clause 24 or of any of clauses 24-34, wherein the guide aperture is a metal material.
36. The surgical system of clause 24 or of any of clauses 24-35, wherein the guide aperture is a radiolucent material.
37. The surgical system of clause 24 or of any of clauses 24-36, wherein the insert further comprises a plurality of radiopaque markers disposed within the insert.
38. The surgical system of clause 24 or of any of clauses 24-37, wherein the first portion and the second portion are two separate parts that are coupled together with two pins.
39. The surgical system of clause 24 or of any of clauses 24-38, wherein the body is generally circular.
40. The surgical system of clause 39, wherein the insert is generally circular.
41. The surgical system of clause 24 or of any of clauses 24-40, wherein the surgical guide has a patient-specific surface.
42. The surgical system of clause 41, wherein the patient-specific surface of the surgical guide is based at least in part on pre-operative imaging of a bone.
43. The surgical system of clause 24 or of any of clauses 24-42, wherein the guide aperture is substantially rectangular, transverse, a wedge shape, or a chevron shape.
44. The surgical system of clause 24 or of any of clauses 24-43, wherein the surgical guide further comprises a handle configured to remove the surgical guide from a bone.
45. The surgical system of clause 24 or of any of clauses 24-44, wherein the surgical navigation system further comprises an imaging device communicatively coupled to the computing device configured to track a change in position of the insert and transmit the position of the insert to the computing device to be displayed on the display.
46. The surgical system of clause 45, wherein the imaging device is configured to track the position of the insert by tracking a movement of a plurality of radiopaque markers disposed within the insert as the insert is rotated.
47. The surgical system of clause 24 or of any of clauses 24-46, wherein the surgical navigation system is referenced based on an initial placement of the surgical guide on a bone with a pre-determined size.
48. The surgical system of clause 24 or of any of clauses 24-47, wherein the insert defines one or more holding features configured to receive a fixation element.
49. The surgical system of clause 24 or of any of clauses 24-48, wherein the insert defines an aperture that is sized and configured to receive a sight and an alignment ring.
50. A method of using a surgical guide comprising:
   coupling the surgical guide to a bone with one or more first fixation elements;
   adjusting, to a first angle, an insert disposed within a body of the surgical guide to align a guide aperture with a desired surgical site on the bone;
   locking the insert;
   inserting a surgical instrument into the guide aperture; and
   making a first cut of the bone with the surgical instrument.
51. The method of clause 50, further comprising unlocking the insert, adjusting the insert to a second angle, and re-locking the insert at the second angle.
52. The method of clause 51, further comprising making a second cut of the bone with the surgical instrument.
53. The method of clause 51, wherein the first angle and the second angle are referenced to the bone and are determined based at least in part on at least one of a pre-operative plan, a post-operative plan, or intraoperative decision.
54. The method of clause 50 or of any of clauses 50-53, further comprising aligning the surgical guide, facilitated by a sight and an alignment ring disposed within an aperture defined by the insert.
55. A method of using a surgical system comprising:
   determining a reference point for a surgical procedure based at least in part on a surgical navigation system;
   coupling a surgical guide to a bone with one or more first fixation elements;
   adjusting, to a first angle, an insert disposed within the surgical guide to align a guide aperture with a desired surgical site on the bone based at least in part on the surgical navigation system;
   locking the insert;
   inserting a surgical instrument into the guide aperture; and
   making a first cut of the bone with the surgical instrument.
56. The method of clause 55, further comprising unlocking the insert, adjusting the insert to a second angle determined based at least in part by the surgical navigation system, and re-locking the insert at the second angle.
57. The method of clause 56, further comprising making a second cut of the bone with the surgical instrument.
58. The method of clause 57, wherein the first angle and the second angle are referenced to the bone and determined based at least in part on at least one of a pre-operative plan, a post-operative plan, or intraoperative decision.
59. The method of clause 55 or of any of clauses 55-58, wherein the determining step is based at least in part on a database of bones.
60. The method of clause 55 or of any of clauses 55-59, further comprising displaying the adjustment of the insert on a display.
61. The method of clause 55 or of any of clauses 55-60, wherein a display is configured to display at least one of a pre-operative plan, a post-operative plan, and an intraoperative adjustment of the guide aperture based at least in part on a desired shape of a procedure.
62. The method of clause 55 or of any of clauses 55-61, further comprising aligning the surgical guide, facilitated by a sight and an alignment ring disposed within an aperture defined by the insert.
63. A surgical guide, comprising:
   a body having a first portion with a first ridge and a second ridge, and a second portion with a first ridge and a second ridge;
   an insert having a raised portion, wherein the insert is disposed between the first ridge and the second ridge of the first portion and the first ridge and second ridge of the second portion, and wherein the insert is configured to rotate relative to the body; and
   a guide aperture disposed within the insert that is sized to receive a surgical instrument.
64. The surgical guide of clause 63, wherein the body includes a plurality of first protrusions and a plurality of second protrusions that define a plurality of channels configured to allow one or more fixation elements to pass through the body into a bone.
65. The surgical guide of clause 64, wherein the one or more fixation elements are k-wires.
66. The surgical guide of clause 63 or of any of clauses 63-65, wherein the surgical guide further comprises an irrigation port configured to provide irrigation to a surgical site.
67. The surgical guide of clause 63 or of any of clauses 63-66, wherein the insert further comprises one or more holes disposed through the insert configured to allow one or more fixation elements to pass through the insert and into a bone.
68. The surgical guide of clause 63 or of any of clauses 63-67, wherein the insert is locked with a locking device.
69. The surgical guide of clause 63 or of any of clauses 63-68, the surgical guide further comprises a plurality of indicia configured to indicate a change in orientation of the guide aperture as the insert is rotated.
70. The surgical guide of clause 69, wherein the insert further comprises an indicator disposed on the insert configured to point to one of the plurality of the indicia.
71. The surgical guide of clause 63 or of any of clauses 63-70, wherein the insert is rotatable in 360 degrees with respect to the body.
72. The surgical guide of clause 63 or of any of clauses 63-71, wherein the guide aperture is a slot configured to receive the surgical instrument.
73. The surgical guide of clause 63 or of any of clauses 63-72, wherein the guide aperture is an opening configured to receive the surgical instrument.
74. The surgical guide of clause 63 or of any of clauses 63-73, wherein the guide aperture is a metal material.
75. The surgical guide of clause 63 or of any of clauses 63-74, wherein the guide aperture is a radiolucent material.
76. The surgical guide of clause 63 or of any of clauses 63-75, wherein the insert further comprises a plurality of radiopaque markers disposed within the insert.
77. The surgical guide of clause 63 or of any of clauses 63-76, wherein the first portion and the second portion are two separate parts that are coupled together with two pins.
78. The surgical guide of clause 63 or of any of clauses 63-77, wherein the body is generally circular.
79. The surgical guide of clause 78, wherein the insert is generally circular.
80. The surgical guide of clause 63 or of any of clauses 63-79, wherein the surgical guide has a patient-specific surface.
81. The surgical guide of clause 80, wherein the patient-specific surface of the surgical guide is based at least in part on pre-operative imaging of a bone.
82. The surgical guide of clause 63 or of any of clauses 63-81, wherein the guide aperture is substantially rectangular, transverse, a wedge shape, or a chevron shape.
83. The surgical guide of clause 63 or of any of clauses 63-82, further comprising a handle configured to remove the surgical guide from a bone.
84. The surgical guide of clause 63 or of any of clauses 63-83, wherein the insert defines one or more holding features configured to receive a fixation element.
85. The surgical guide of clause 63 or of any of clauses 63-84, wherein the insert defines an aperture that is sized and configured to receive a sight and an alignment ring.
86. A surgical guide comprising:
   a body having a raised portion, the body defining:
      one or more holding features configured to receive a fixation element; and
      an aperture that is sized and configured to receive a sight and an alignment ring;
   a guide aperture disposed within the body that is sized to receive a surgical instrument; and
   an irrigation port configured to provide irrigation to a surgical site.
87. The surgical guide of clause 86, wherein the body further comprises one or more channels disposed through the body configured to allow one or more fixation elements to pass through the insert and into a bone.
88. The surgical guide of clause 86 or of any of clauses 86-87, wherein the body further comprises a ring to facilitate rotation of the body with respect to a second surgical guide.
89. The surgical guide of clause 86 or of any of clauses 86-88, wherein the guide aperture is a slot configured to receive the surgical instrument.
90. The surgical guide of clause 86 or of any of clauses 86-89, wherein the guide aperture is an opening configured to receive the surgical instrument.
91. The surgical guide of clause 86 or of any of clauses 86-90, wherein the guide aperture is a metal material.
92. The surgical guide of clause 86 or of any of clauses 86-91, wherein the guide aperture is a radiolucent material.
93. The surgical guide of clause 86 or of any of clauses 86-92, wherein the body further comprises a plurality of radiopaque markers disposed within the insert.
94. The surgical guide of clause 86 or of any of clauses 86-93, wherein the body is substantially rectangular.
95. The surgical guide of clause 86 or of any of clauses 86-94, wherein the body is substantially oblong.
96. The surgical guide of clause 86 or of any of clauses 86-95, wherein the surgical guide has a patient-specific surface.
97. The surgical guide of clause 96, wherein the patient-specific surface of the surgical guide is based at least in part on pre-operative imaging of a bone.
98. The surgical guide of clause 86 or of any of clauses 86-97, wherein the guide aperture is substantially transverse, a wedge shape, or a chevron shape.
99. The surgical guide of clause 86 or of any of clauses 86-98, further comprising a handle configured to remove the surgical guide from a bone.

## Claims

1. A surgical guide, comprising:
a body having a first portion with a first ridge and a second ridge, and a second portion with a first ridge and a second ridge;
an insert disposed between the first ridge and the second ridge of the first portion and the first ridge and second ridge of the second portion, wherein the insert is configured to rotate relative to the body; and
a guide aperture disposed within the insert that is sized to receive a surgical instrument.

2. The surgical guide of claim 1, wherein the body includes a plurality of first protrusions and a plurality of second protrusions that define a plurality of channels configured to allow one or more fixation elements to pass through the body into a bone, wherein optionally the one or more fixation elements are k-wires.

3. The surgical guide of any of the preceding claims, wherein the surgical guide further comprises an irrigation port configured to provide irrigation to a surgical site, and/or wherein the insert further comprises one or more holes disposed through the insert configured to allow one or more fixation elements to pass through the insert and into a bone, and/or wherein the insert is locked with a locking device.

4. The surgical guide of any of the preceding claims, wherein the surgical guide further comprises a plurality of indicia configured to indicate a change in orientation of the guide aperture as the insert is rotated, wherein the insert optionally further comprises an indicator disposed on the insert configured to point to one of the plurality of the indicia.

5. The surgical guide of any of the preceding claims, wherein the insert is rotatable in 360 degrees with respect to the body.

6. The surgical guide of any of the preceding claims, wherein the guide aperture is a slot configured to receive the surgical instrument, and/or wherein the guide aperture is an opening configured to receive the surgical instrument, and/or wherein the guide aperture is a metal material, and/or wherein the guide aperture is a radiolucent material.

7. The surgical guide of any of the preceding claims, wherein the insert further comprises a plurality of radiopaque markers disposed within the insert.

8. The surgical guide of any of the preceding claims, wherein the first portion and the second portion are two separate parts that are coupled together with two pins.

9. The surgical guide of any of the preceding claims, wherein the body is generally circular, wherein optionally the insert is generally circular.

10. The surgical guide of any of the preceding claims, wherein the surgical guide has a patient-specific surface, wherein optionally the patient-specific surface of the surgical guide is based at least in part on pre-operative imaging of a bone.

11. The surgical guide of any of the preceding claims, wherein the guide aperture is substantially rectangular, transverse, a wedge shape, or a chevron shape, and/or the surgical guide further comprising a handle configured to remove the surgical guide from a bone, and/or wherein the insert defines one or more holding features configured to receive a fixation element, and/or wherein the insert defines an aperture that is sized and configured to receive a sight and an alignment ring.

12. A surgical system comprising:
the surgical guide of any of the preceding claims; and
a surgical navigation system having a computing device with a processor and a display configured to display intraoperative adjustments of the guide aperture.

13. The surgical system of claim 12, wherein the surgical navigation system further comprises an imaging device communicatively coupled to the computing device configured to track a change in position of the insert and transmit the position of the insert to the computing device to be displayed on the display, wherein optionally the imaging device is configured to track the position of the insert by tracking a movement of a plurality of radiopaque markers disposed within the insert as the insert is rotated.

14. The surgical system of any of claims 12-13, wherein the surgical navigation system is referenced based on an initial placement of the surgical guide on a bone with a pre-determined size.

15. The surgical system of any of claims 12-14, wherein the insert defines one or more holding features configured to receive a fixation element, and/or wherein the insert defines an aperture that is sized and configured to receive a sight and an alignment ring.
